# EUROPEAN PATENT APPLICATION

(11) **EP 4 691 485 A1**
(43) Date of publication of application: **11.02.2026**
(21) Application number: 24780521.1
(22) Date of filing: 27.03.2024
(51) Int. Cl.: A61K 39/39, A61K 9/08, A61K 39/00, A61K 39/002, A61K 39/02, A61K 39/12, A61K 39/35, A61K 39/145, A61K 39/215, A61P 31/00, A61P 31/04, A61P 31/10, A61P 31/12, A61P 31/14, A61P 31/16, A61P 33/00, A61P 37/04, C07D 473/16

(54) **MUCOSAL ADJUVANT**

(30) Priority: 31.03.2023 JP 2023057218
(71) Applicant: Toyama Prefecture, Toyama-shi, Toyama 930-8501 (JP); Toyama Prefectural University, Imizu-shi, Toyama, 939-0398 (JP)
(72) Inventor: TAKATSU, Kiyoshi, Imizu-shi, Toyama 939-0363 (JP); AIKAWA, Yukihiko, Imizu-shi, Toyama 939-0363 (JP); WATANABE, Yasuharu, Imizu-shi, Toyama 939-0363 (JP); YANAGIBASHI, Tsutomu, Imizu-shi, Toyama 939-0363 (JP); MINAMITANI, Takeharu, Imizu-shi, Toyama 939-0363 (JP); HONDA, Hiroe, Imizu-shi, Toyama 939-0363 (JP); MIYAMOTO, Tomomi, Kurobe-shi, Toyama 938-0025 (JP); NAGAI, Yoshinori, Imizu-shi, Toyama 939-0398 (JP)
(74) Representative: Vossius & Partner Patentanwälte Rechtsanwälte mbB
(86) International application number: PCT/JP2024/012399
(87) International publication number: WO 2024/204419

(57) **Abstract**

The present invention provides a mucosal adjuvant comprising a compound represented by Formula (I) A-L-B, wherein A represents a structure having TLR7 agonist activity, L represents a linker, and B represents a structure having TLR2 agonist activity, or a pharmaceutically acceptable salt thereof.

## Description

### Technical Field

The present invention relates to a mucosal adjuvant, a composition comprising the same, and a novel compound used for the same.

### Background Art

Vaccination is the most effective means for preventing infectious diseases, including influenza. However, existing subcutaneous influenza vaccines induce IgG antibodies involved in systemic immunity in the blood, but cannot induce secretory IgA antibodies, which play an important role in preventing viral infection in mucous immunity, in the mucous membranes. Therefore, the expected effect of existing vaccines is not the prevention of infection, but the prevention of progression of the disease.

Mucosal immunity is important for host defense against natural viral infections, and the mechanisms of viral recognition, interferon induction, and secretory IgA antibody production at the mucous membranes are being elucidated. In particular, secretory IgA antibodies secreted in mucosal tissues are known to play a very important role in preventing respiratory infections such as influenza. This is because secretory IgA antibodies can neutralize pathogens at the mucous membranes before infection is established in the host. In addition, IgA antibodies have a cross-protective ability and can prevent infection even against different viral subtypes. For influenza vaccines, types expected to be prevalent in that year are predicted and vaccines are manufactured. However, with existing vaccines, if the prevalent strain differs from the vaccine strain, their effectiveness significantly decreases, and in some cases, there is almost no effect. From these facts, there is a high demand for vaccine development that enables infection prevention by efficiently inducing the production of secretory IgA antibodies through the activation of mucosal immunity, in addition to inducing systemic immune responses by IgG antibodies. The utility of mucosal vaccines capable of efficiently inducing IgA antibodies in mucosal tissues has attracted attention. However, mucosal administration of the same components as subcutaneously administered vaccines hardly induces mucosal immunity, and secretory IgA antibodies are rarely induced. Therefore, in addition to conventional vaccine components, substances that induce mucosal immunity, i.e., adjuvants for vaccines that induce mucosal immunity, are needed, and desired to be developed.

As known examples of subcutaneous vaccine adjuvants, those containing Toll-like receptor (TLR) agonists are known, and the following reports have been made.

Patent Documents 1 and 2 describe compounds that are covalently conjugated TLR7 and/or TLR8 and TLR2 agonists, used for inducing innate immune responses. The working examples show the anti-tumor effect of the compounds in a mouse model. However, the use examples of the compounds in Patent Documents 1 and 2 are for administration to subcutaneously injected tumors, and administration to mucous membranes is not taught. Furthermore, the production of IgA antibodies at mucous membranes is not described. CL553 described in Patent Document 1 is used as a racemate.

Non-Patent Document 1 describes that a dual TLR2 and TLR7 ligand induces humoral and cellular immune responses. Experiments show an increase in serum IgG antibody titers by administering a dual TLR2 and TLR7 ligand with an antigen protein to mice. However, the use example of the compound in Non-Patent Document 1 is for subcutaneous administration, and mucosal administration is not taught. Furthermore, it is described that subcutaneous administration does not induce IgA antibody production in mucous membranes. PamadiFectin^{®} described in Non-Patent Document 1 is used as a racemate.

### Prior Art Documents

### Patent Document

[Patent Document 1] EP2769738
[Patent Document 2] EP2732825

### Non-Patent Document

Non-Patent Document 1: J Immunol 2017;198:4205. A Dual TLR2 and TLR7 Ligand Induces Highly Potent Humoral and Cell-Mediated Immune Responses

### Summary of Invention

### Problem to be Solved by the Invention

In view of the above circumstances, the present invention aims to provide a mucosal adjuvant capable of inducing IgA antibodies in mucous membranes, a composition comprising the same, and a novel compound used for the same.

### Means for Solving the Problems

The inventors of the present invention diligently studied substances capable of exhibiting an effect as a mucosal adjuvant, and as a result, found a specific compound capable of inducing IgA antibodies in mucous membranes as a mucosal adjuvant, thereby completed the present invention.

That is, the gist of the present invention is as follows:
[1] A mucosal adjuvant comprising a compound represented by the following formula (I) or a pharmaceutically acceptable salt thereof:

   A-L-B (I)

   wherein,
   A represents a structure having TLR7 agonist activity,
   L represents a linker, and
   B represents a structure having TLR2 agonist activity.
   Further, as another aspect of the present invention, a mucosal adjuvant formulation comprising a compound represented by formula (I) or a pharmaceutically acceptable salt thereof is provided.
   Further, as another aspect of the present invention, the use of a compound represented by formula (I) or a pharmaceutically acceptable salt thereof in the manufacture of a mucosal adjuvant formulation is provided.
   Further, as another aspect of the present invention, a compound represented by formula (I) or a pharmaceutically acceptable salt thereof, for use as a mucosal adjuvant formulation, is provided.
[2] The adjuvant according to [1], wherein L has a structure represented by the following formula (L-1): 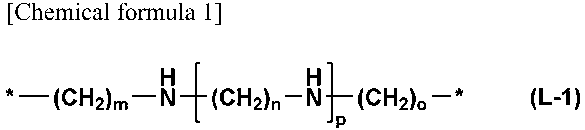 wherein,
   m represents an integer from 0 to 4,
   n represents an integer from 0 to 4,
   o represents an integer from 0 to 4,
   p represents an integer from 1 to 6, and
   * indicates a bond to -NH-.
[3] The adjuvant according to [1] or [2], wherein the compound represented by formula (I) is compound TY001 represented by the following formula. 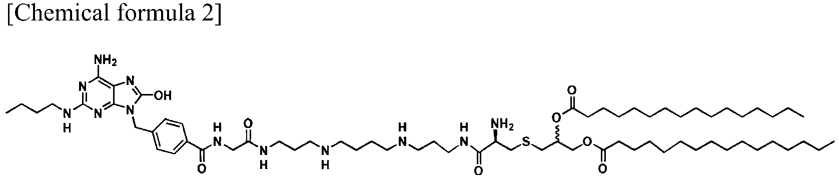
[4] The adjuvant according to [3], wherein TY001 is a racemate, S-form, or R-form.
[5] A vaccine composition comprising at least one antigen and the adjuvant according to any one of [1] to [4].
   Further, as another aspect of the present invention, the use of at least one antigen and the adjuvant according to any one of [1] to [4] in the manufacture of a vaccine composition is provided.
   Further, as another aspect of the present invention, at least one antigen and the adjuvant according to any one of [1] to [4], for use as a vaccine composition, are provided.
   Further, as another aspect of the present invention, a method for enhancing immunity in a subject, comprising the step of administering at least one antigen and the adjuvant according to any one of [1] to [4] to the subject, is provided.
[6] The composition according to [5], further comprising chondroitin sulfate and/or a salt thereof.
[7] The composition according to [5] or [6], which is a liquid formulation, a spray formulation, a semi-solid formulation, or a solid formulation, wherein the semi-solid formulation and the solid formulation dissolve in body fluids and/or at body temperature.
[8] The composition according to any one of [5] to [7], for use in inducing humoral immunity.
[9] The composition according to any one of [5] to [8], for use in inducing the production of IgA antibodies in mucous membranes.
[10] The composition according to [9], further for use in inducing the production of IgG antibodies in the blood.
[11] The composition according to any one of [5] to [10], which is a vaccine for infectious diseases.
[12] The composition according to [11], wherein the antigen of the vaccine for infectious diseases is an antigen derived from a pathogenic virus, pathogenic bacterium, pathogenic fungus, or parasite.
[13] The composition according to [12], wherein the antigen of the vaccine for infectious diseases is an antigen derived from influenza virus or coronavirus.
[14] The composition according to any one of [5] to [10], which is a vaccine for non-infectious diseases.
[15] The composition according to [14], wherein the antigen of the vaccine for non-infectious diseases is an antigen derived from amyloid β, α-synuclein, prion, cholesteryl ester transfer protein, ApoB100, oxidized LDL, angiotensin I/II, glatiramer acetate, myelin basic protein, T cell receptor of MBP-specific T cells, insulin, GAD, T cell receptor of acetylcholine receptor-specific T cells, allergen, IL-5, cancer antigen, neoantigen, intoxicating substance, TNFα, HCG, GnRH, Ghrelin, or TRANCE/RANKL.
[16] A compound represented by the following formula, or a pharmaceutically acceptable salt thereof. 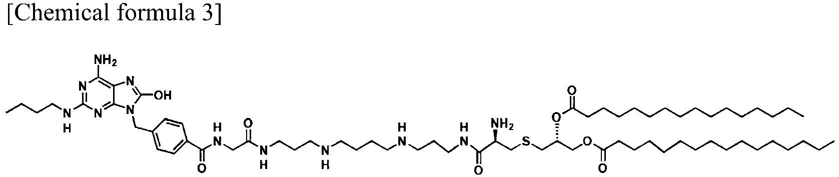
[17] A compound represented by the following formula, or a pharmaceutically acceptable salt thereof.

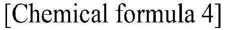

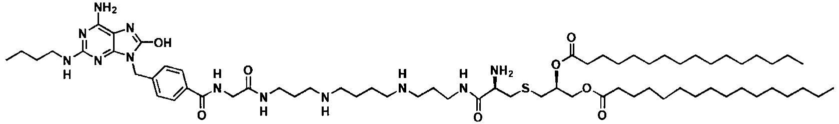

### Advantageous Effects of Invention

The present invention provides a mucosal adjuvant capable of inducing IgA antibodies in mucous membranes, a composition comprising the same, and a novel compound used for the same.

### Brief Description of Drawings

[Fig. 1] Fig. 1 is a diagram showing the virus infectivity titer in nasal lavage fluid when TY001 is used as a mucosal adjuvant (Example 1).
[Fig. 2] Fig. 2 is a diagram showing the level of antigen-specific IgA antibody in nasal lavage fluid (2²-fold dilution) when TY001 is used as a mucosal adjuvant (Example 1).
[Fig. 3] Fig. 3 is a diagram showing the level of antigen-specific IgG antibody in serum (2¹²-fold dilution) when TY001 is used as a mucosal adjuvant (Example 1).
[Fig. 4] Fig. 4 is a diagram showing the level of antigen-specific IgA antibody in nasal lavage fluid and antigen-specific IgG antibody in plasma when CL401, CL413, and CL531 are used as mucosal adjuvants (Example 2).
[Fig. 5] Fig. 5 is a diagram showing the level of antigen-specific IgA antibody in nasal lavage fluid (2³-fold dilution) when TY001 S-form is used as a mucosal adjuvant (Example 3).
[Fig. 6] Fig. 6 is a diagram showing the level of antigen-specific IgG antibody in serum (2⁸-fold dilution) when TY001 S-form is used as a mucosal adjuvant (Example 3).
[Fig. 7] Fig. 7 is a diagram showing the level of antigen-specific IgA antibody in nasal lavage fluid and antigen-specific IgG antibody in serum when TY001 S-form is used as a mucosal adjuvant (Example 4).
[Fig. 8] Fig. 8 is a diagram showing the virus infectivity titer in nasal lavage fluid when TY001 S-form is used as a mucosal adjuvant (Example 5).
[Fig. 9] Fig. 9 is a diagram showing the level of antigen-specific IgA antibody in nasal lavage fluid and antigen-specific IgG antibody in serum when TY001 S-form is used as a mucosal adjuvant (Example 5).
[Fig. 10] Fig. 10 is a diagram showing the level of antigen-specific IgA antibody in nasal lavage fluid and bronchoalveolar lavage fluid, and antigen-specific IgG antibody in serum and bronchoalveolar lavage fluid when TY001 S-form is used as a mucosal adjuvant (Example 6).

### Embodiments for Carrying Out the Invention

Hereinafter, the embodiments of the present invention will be described. However, the present invention is not limited to the following preferred embodiments and can be freely modified within the scope of the present invention. In this specification, for numerical ranges expressed as "lower limit to upper limit," the upper limit may be "or less" or "less than," and the lower limit may be "or more" or "greater than."

### <Definitions>

In this specification, the terms "vaccine" and "vaccine composition" are used interchangeably and mean a composition comprising an antigen and an adjuvant that induces an immune response when administered into the body of an animal such as a human. A vaccine may be a drug for preventing or treating infectious diseases or non-infectious diseases in animals such as humans, and a vaccine for preventing infectious diseases or non-infectious diseases in animals such as humans may be referred to as a "preventive vaccine," and a vaccine for treating infectious diseases or non-infectious diseases in animals such as humans may be referred to as a "therapeutic vaccine."

"Antigen" (also referred to as antigenic substance or immunogen) in this specification refers to a substance included in a vaccine that induces an immune response when administered into the body of an animal such as a human (this property is referred to as "having antigenicity"). In this specification, unless it causes inconsistency, the term "antigen" includes nucleic acids that produce substances or parts thereof that are targets of immune responses by transcription/translation in vivo, in addition to substances or parts thereof that are direct targets of immune responses. An antigen may be a natural product or an artificial product (e.g., one artificially produced by genetic recombination or other methods), and may be a microorganism (e.g., pathogenic virus, pathogenic bacterium, pathogenic fungus, parasite, etc.) or the cell itself, or a part thereof. For example, an antigen may be a protein or a part thereof, a peptide, a polysaccharide, which has antigenicity, and a nucleic acid (typically, mRNA or DNA comprising a nucleotide sequence encoding a gene that expresses an antigenic protein or a part thereof), other biological substances or parts thereof, or modified forms thereof. When the antigen is a microorganism, a microorganism having infectivity (live vaccine, attenuated vaccine, etc.) or a microorganism that has lost infectivity (inactivated vaccine, etc.) is used.

When targeting infectious diseases, the antigen is usually derived from a pathogenic virus, pathogenic bacterium, pathogenic fungus, or parasite (e.g., a pathogenic virus, bacterium, pathogenic fungus, or parasite that infects via mucous membranes), and when such an antigen is included, the vaccine of the present invention is used as a vaccine for infectious diseases. Alternatively, when targeting non-infectious diseases, the antigen is usually an antigen other than those derived from viruses, bacteria, fungi, or parasites, for example, substances such as proteins, lipids, or allergens related to the disease, and when such an antigen is included, the vaccine of the present invention is used as a vaccine for non-infectious diseases.

In this specification, an antigen derived from a certain substance or bacterium/virus/pathogenic fungus/parasite may be the substance or bacterium/virus/pathogenic fungus/parasite itself, or an antigenic part of the substance or bacterium/virus/pathogenic fungus/parasite, or a substance containing such a part. Here, the substance or bacterium/virus/pathogenic fungus/parasite itself, and the antigenic part of the substance or bacterium/virus/pathogenic fungus/parasite, may have a natural structure, but may also have artificial modifications. For example, they may include structures not found in nature or have undergone artificial modifications to correspond to multiple viral variants or to enhance antigenicity.

Examples of pathogenic bacteria include, but are not limited to, *Bordetella pertussis, Neisseria meningitidis, Haemophilus influenzae* type b, *Streptococcus pneumoniae, Mycobacterium tuberculosis, Vibrio cholerae, Corynebacterium diphtheriae,* and the like.

Examples of pathogenic viruses include, but are not limited to, varicella virus, measles virus, mumps virus, poliovirus, rotavirus, influenza virus, adenovirus, herpesvirus, RS virus, coronavirus, human immunodeficiency virus (HIV), human papillomavirus, rubella virus, hepatitis virus, etc., and preferably influenza virus, coronavirus, or human immunodeficiency virus, and most preferably influenza virus or coronavirus.

For example, influenza virus antigens are preferably molecules exposed on the surface of the particle, such as hemagglutinin (HA: HA1, HA2), neuraminidase (NA), matrix (M1, M2), non-structural (NS), polymerase (PB1, PB2: basic polymerases 1 and 2, acidic polymerase (PA)), and nucleoprotein (NP). Currently, 16 types of HA and 9 types of NA are known, and any of them can be used as antigens for vaccines of the present invention. In addition, composite antigens of HA and NA, universal antigens, and the like may also be preferably used.

Antigens of coronaviruses include, for example, spike (S), nucleocapsid (N), membrane (M), and envelope (E), as well as proteins encoded by ORF1a, ORF1b, ORF3a, ORF3b, ORF6, ORF7a, ORF7b, ORF8, ORF9b, ORF9c, ORF10, and the like. For specific sequences, reference can be made to, for example, the genomic sequence of SARS coronavirus 2 (accession number LC522975). Coronaviruses include desired viruses belonging to the family Coronaviridae, preferably severe acute respiratory syndrome (SARS) coronavirus or Middle East respiratory syndrome (MERS) coronavirus, and more preferably SARS coronavirus, particularly SARS coronavirus type 1 (SARS-CoV-1), and SARS coronavirus type 2 (SARS-CoV-2), and coronaviruses derived therefrom.

When the targeted disease is a non-infectious disease, the antigen can be appropriately determined from substances whose expression is involved in the onset or exacerbation of the targeted disease, and is not particularly limited, but examples include antigens derived from amyloid beta, alpha-synuclein, prion, cholesterol ester transfer protein, ApoB100, oxidized LDL, angiotensin I/II, glatiramer acetate, myelin basic protein, MBP-specific T cell receptor of T cells, insulin, GAD, acetylcholine receptor-specific T cell receptor of T cells, allergen, IL-5, cancer antigen, neoantigen, intoxicant, TNFa, HCG, GnRH, Ghrelin, or TRANCE/RANKL.

In the present specification, "inactivated antigen" refers to an antigen that has antigenicity but has lost infectivity, used as an antigen, and examples include, but are not limited to, virions which are complete virus particles, incomplete virus particles, virion-like particles, structural or non-structural proteins of viruses, proteins or glycoproteins derived from microorganisms, infection-protective antigens, and substances containing a part of microorganisms that become epitopes of a neutralization reaction. In the present specification, a vaccine containing an inactivated antigen as an antigen is referred to as an "inactivated vaccine." Inactivated antigens can be obtained by inactivating microorganisms through physical operations (e.g., X-ray irradiation, heat, ultrasound) or chemical operations (e.g., formalin, mercury, alcohol, chlorine), but are not limited thereto.

Antigens can be produced by purifying naturally occurring pathogenic viruses, pathogenic bacteria, pathogenic fungi, or parasites and other microorganisms after physical and/or chemical treatment, by artificial synthesis, or by utilizing genetic recombination technology to produce them in microorganisms or cells, or by synthesizing them in a cell-free system. These methods can be carried out using well-known conventional techniques, and commercially available equipment, reagents, and vectors.

When the antigen is a nucleic acid that expresses a protein or a part thereof which is a target of an immune response, its form is not limited, and it may be DNA or RNA. All or part of the nucleotides or nucleosides constituting the nucleic acid may be modified as necessary. For example, pseudouridine or 2-thiouridine may be used instead of uridine, 6-methyladenosine or inosine may be used instead of adenosine, 5-methylcytidine may be used instead of cytidine, or a nucleoside in which the 2' hydroxyl group is replaced with a methoxy group (2'-O-nucleoside) may be used. Furthermore, the nucleic acid may have proliferative capacity, such as self-replicating type (e.g., self-replicating mRNA) or trans-replicating type (e.g., trans-replicating mRNA). For nucleic acids, desired vectors such as naked nucleic acids, plasmids, viral vectors, non-viral vectors (e.g., liposomes) can be used. In the case of DNA, DNA having a nucleic acid encoding a protein or a portion thereof that is a target for immune response under the control of any promoter that is activated in the host can be used. Examples of such promoters include, but are not limited to, CMV promoter, CAG promoter, SV40 promoter, RSV promoter, EF1α promoter, SRα promoter, and the like.

In the case of antigens derived from pathogenic viruses, pathogenic bacteria, pathogenic fungi, or parasites, from a safety perspective, inactivated antigens or nucleic acids encoding a gene that expresses the antigen are desirable.

In this specification, "mucosal administration" refers to an administration form via mucous membranes. In this specification, "mucous membranes" refers to the inner wall of hollow organs, especially those communicating externally, such as the digestive, respiratory, and urogenital tracts in vertebrates. Therefore, examples of such mucosal administration include, but are not limited to, nasal administration (intranasal administration), oral administration, vaginal administration, upper respiratory tract administration, and alveolar administration (pulmonary administration). Preferably, nasal administration or oral administration is advantageous. In particular, nasal administration allows for early defense against infection because the nasal cavity is an infection route for respiratory infectious diseases such as influenza virus and coronavirus.

In this specification, "adjuvant" refers to a substance that activates immunity and includes substances used to increase or induce an immune response to an administered antigen. "Mucosal adjuvant" refers to a substance that activates local and/or systemic immunity at the mucous membranes when administered mucosally. A preferable mucosal adjuvant is a substance that enhances IgA production locally at the mucous membranes and/or increases IgG levels in the blood.

### <Mucosal Adjuvant and Vaccine>

One aspect of the present invention relates to a mucosal adjuvant (hereinafter sometimes referred to as "the adjuvant of the present invention") comprising a compound represented by the following formula (I) or a pharmaceutically acceptable salt thereof:

A-L-B (I)

wherein,
A represents a structure having TLR7 agonist activity,
L represents a linker, and
B represents a structure having TLR2 agonist activity.

The present inventors found that a dual agonist of TLR2 and TLR7, i.e., the compound represented by the above formula (I), is excellent as a mucosal adjuvant capable of inducing secretory IgA antibodies on mucous membranes. Based on this finding, they discovered that a vaccine containing the compound represented by the above formula (I) or a pharmaceutically acceptable salt thereof as a mucosal adjuvant is excellent in inducing immunity against a target antigen. The present invention was thus completed. The adjuvant of the present invention can, for example, induce IgA antibodies against the antigen in mucous membranes when administered mucosally together with the antigen, and preferably can further induce IgG antibodies against the antigen in the blood. One aspect of the present invention is an adjuvant for mucosal immune induction. Another aspect of the present invention is an adjuvant for mucosal administration.

In the above formula (I), A is not limited as long as it has TLR7 agonist activity. A is not limited to, but may be, for example, a structure derived from: a purine base (adenine, guanine, etc.) or an analog or derivative thereof; a pyrimidine base (cytosine, thymine, uracil, etc.) or an analog or derivative thereof; and an imidazoquinoline or an analog or derivative thereof. It may also include structures derived from multiple compounds. Furthermore, A may be a structure included in specific examples of compounds represented by the general formula (I) described below. Derivatives are not limited to, but may include, for example, nucleosides (guanosine, etc.) or structures having a substituent on the purine ring in a purine base, the pyrimidine ring in a pyrimidine base, or the imidazoquinoline ring in imidazoquinoline. Substituents are not limited to, but may include, for example, hydroxy, amino, C1-6 alkylamino, C1-6 alkyl, C1-6 alkoxy, or C1-6 acyl. Any carbon atom contained in alkylamino, alkyl, alkoxy, or acyl may be substituted with one or more heteroatoms selected from nitrogen, oxygen, and sulfur atoms. In addition, alkylamino, alkyl, alkoxy, or acyl may be substituted with hydroxy, amino, or the like. Furthermore, preferred substituents are hydroxy, amino, C1-4 alkylamino which may be substituted with a heteroatom, and C1-4 alkyl which may be substituted with a heteroatom. A may further have substituents as long as they do not affect the effect of the present invention.

B is not limited as long as it has TLR2 agonist activity. B is not limited to, but may be, for example, a structure derived from: a lipopeptide (monoacylated lipopeptide such as PamCys (palmitoyl-S-glycerylcysteine), diacylated lipopeptide such as Pam2Cys (dipalmitoyl-S-glycerylcysteine), or triacylated lipopeptide such as Pam3Cys (tripalmitoyl-S-glycerylcysteine)) or a derivative thereof (PamCSK4, Pam2CSK4, Pam3CSK4, etc.). It may also include structures derived from multiple compounds. Furthermore, B may be a structure included in specific examples of compounds represented by the general formula (I) described below. B may have substituents that do not affect the effect of the present invention. Specific examples of substituents are the same as those described for A.

L represents a linker, which connects A and B, and is not limited as long as it can achieve the effects of the present invention. The linker connects A and B, and may be absent if the structure derived from A and/or B can sufficiently secure the necessary distance between A and B to achieve the effects of the present invention. The linker typically includes any structure necessary for binding with A and B. L may have substituents that do not affect the effects of the present invention. Specific examples of substituents are the same as those described for A.

Furthermore, the compound represented by formula (I) used as the adjuvant of the present invention does not preclude the inclusion of structures other than A, B, and L that do not affect the effects of the present invention within the compound's structure.

Specific examples of compounds represented by the above general formula (I) include, but are not limited to, the compounds listed below:
(20R)-20-amino-1-(4-((6-amino-2-(butylamino)-8-hydroxy-9H-purin-9-yl)methyl) phenyl)-1,4, 19-trioxo-22-thia-2,5,9,14,18-pentaazapentacosane-24,25-diyl dipalmitate; (20R)-1-(4-((6-amino-2-(butylamino)-8-hydroxy-9H-purin-9-yl)methyl)phenyl)-1,4,19-trioxo-20-palmitamido-22-thia-2,5,9,14,18-pentaazapentacosane-24,25-diyl dipalmitate; (6R,9S)-6,23-diamino-9-((4-((6-amino-2-(butylamino)-8-hydroxy-9H-purin-9-yl) methyl)benzoyloxy)methyl)-7,10-dioxo-4-thia-8,11,15,20-tetraazatricosane-1,2-diyl dipalmitate;
(6R,13S)-6,27-diamino-13-(4-((6-amino-2-(butylamino)-8-hydroxy-9H-purin-9-yl) methyl)benzamido)-7,14-dioxo-4-thia-8,15,19,24-tetraazaheptacosane-1,2-diyl dipalmitate;
(6R,13S)-6,27-diamino-13-(2-(4-((6-amino-2-(butylamino)-8-hydroxy-9H-purin-9-yl)methyl) benzamido)acetamido)-7,14-dioxo-4-thia-8,15,19,24-tetraazaheptacosane-1,2-diyl dipalmitate;
(6R,9S)-6,23-diamino-9-(4-(2-(4-((6-amino-2-(butylamino)-8-hydroxy-9H-purin-9-yl)methyl) benzamido)acetamido)butyl)-7,10-dioxo-4-thia-8,11,15,20-tetraazatricosane-1,2-diyl dipalmitate;
(6R,9S)-6,25-diamino-9-(4-((6-amino-2-(butylamino)-8-hydroxy-9H-purin-9-yl) methyl)phenyl carbamoyl)-7,12-dioxo-4-thia-8,13,17,22-tetraazapentacosane-1,2-diyl dipalmitate;
(6S,9S,12S,15S,18S,21R)-6,25-diamino-21-(4-((6-amino-2-(butylamino)-8-hydroxy-9H-purin-9-yl)methyl)phenylcarbamoyl)-12,15,18-tris(4-aminobutyl)-9-(hydroxy methyl)-7,10, 13,16,19-pentaoxo-4-thia-8,11,14,17,20-pentaazapentacosane-1,2-diyl dipalmitate;
(2S,5S,8S,11S,14S,17R)-17-amino-5-(4-(4-((6-amino-2-(butylamino)-8-hydroxy-9H-purin -9-yl)methyl)benzamido)butyl)-2,8,11-tris(4-aminobutyl)-14-(hydroxymethyl)-4,7,10,13, 16,24-hexaoxo-21-(palmitoyloxy)-23-oxa-19-thia-3,6,9,12,15-pentaazanona triacontan-1-oic acid;
(2S,5S,8S,11S,14S,17R)-17-amino-5-(4-(4-((6-amino-2-(butylamino)-8-hydroxy-9H-purin -9-yl)methyl)benzamido)butyl)-2,8,11-tris(4-aminobutyl)-14-(hydroxymethyl)-4,7,10,13, 16,24-hexaoxo-21-(palmitoyloxy)-23-oxa-19-thia-3,6,9,12,15-pentaazanona triacontan-1-oic acid;
(2S,5S,8S,11S,14S,17R)-17-amino-11-(4-(4-((6-amino-2-(butylamino)-8-hydroxy-9H-purin-9-yl)methyl)benzamido)butyl)-2,5,8-tris(4-aminobutyl)-14-(hydroxymethyl)-4,7,10,13,16,24-hexaoxo-21-(palmitoyloxy)-23-oxa-19-thia-3,6,9,12,15-pentaazanona triacontan-1-oic acid;
((6R,9S,12S,15S,18S,21S)-25-amino-21-(4-((6-amino-2-(butylamino)-8-hydroxy-9H-purin-9-yl)methyl)phenylcarbamoyl)-12,15,18-tris(4-aminobutyl)-9-(hydroxymethyl)-7,10,13,16,19-pentaoxo-6-palmitamido-4-thia-8,11,14,17,20-pentaazapentacosane-1,2-diyl dipalmitate;
(2S,5S,8S,11S,14S,17R)-5-(4-(4-((6-amino-2-(butylamino)-8-hydroxy-9H-purin-9-yl)methyl) benzamido)butyl)-2,8,11-tris(4-aminobutyl)-14-(hydroxymethyl)-4,7,10,13, 16,24-hexaoxo-17-palmitamido-21-(palmitoyloxy)-23-oxa-19-thia-3,6,9,12,15-penta azanonatriacontan-1-oic acid;
(2S,5S,8S,11S,14S,17R)-8-(4-(4-((6-amino-2-(butylamino)-8-hydroxy-9H-purin-9-yl)methyl) benzamido)butyl)-2,5,11-tris(4-aminobutyl)-14-(hydroxymethyl)-4,7,10,13, 16,24-hexaoxo-17-palmitamido-21-(palmitoyloxy)-23-oxa-19-thia-3,6,9,12,15-penta azanonatriacontan-1-oic acid;
(2S,5S,8S,11S,14S,17R)-11-(4-(4-((6-amino-2-(butylamino)-8-hydroxy-9H-purin-9-yl) methyl)benzamido)butyl)-2,5,8-tris(4-aminobutyl)-14-(hydroxymethyl)-4,7,10,13,16,24-hexaoxo-17-palmitamido-21-(palmitoyloxy)-23-oxa-19-thia-3,6,9,12,15-penta azanonatria contan-1-oic acid;
(S)-methyl 1-(4-((6-amino-2-(butylamino)-8-hydroxy-9H-purin-9-yl)methyl)phenyl)-22-((R)-2-amino-3-(2-(tetradecanoyloxy)ethylthio)propanamido)-1,4,19-trioxo-2,5,9,14,18-pentaazatricosan-23-oate.
(R)-3-(2-amino-3-(4-((6-amino-2-(butylamino)-8-hydroxy-9H-purin-9-yl)methyl)phenyl amino)-3-oxopropylthio)propane-1,2-diyl dipalmitate;
(R)-3-(2-amino-3-(3-(4-amino-2-(ethoxymethyl)-1H-imidazo[4,5-c]quinolin-1-yl)propyl amino)-3-oxopropylthio)propane-1,2-diyl dipalmitate;
(R)-3-(2-amino-3-(3-(4-amino-1H-imidazo[4,5-c]quinolin-1-yl)propylamino)-3-oxopropylthio) propane-1,2-diyl dipalmitate;
(R)-3-(2-amino-3-(3-(4-amino-2-((ethylamino)methyl)-1H-imidazo[4,5-c]quinolin-1-yl)propyl amino)-3-oxopropylthio)propane-1,2-diyl dipalmitate;
(R)-3-(2-amino-3-(4-((4-amino-2-butyl-1H-imidazo[4,5-c]quinolin-1-yl)methyl)phenyl amino)-3-oxopropylthio)propane-1,2-diyl dipalmitate;
3-((R)-2-amino-3-((S)-1-(4-((6-amino-2-(butylamino)-8-hydroxy-9H-purin-9-yl)methyl) phenylamino)-3-hydroxy-1-oxopropan-2-ylamino)-3-oxopropylthio)propane-1,2-diyl dipalmitate;
(R)-3-(3-(4-((6-amino-2-(butylamino)-8-hydroxy-9H-purin-9-yl)methyl)phenylamino)-3-oxo-2-palmitamidopropylthio)propane-1,2-diyl dipalmitate;
(R)-2-(2-amino-3-(4-((6-amino-2-(butylamino)-8-hydroxy-9H-purin-9-yl)methyl)phenylamino)-3-oxopropylthio)ethyl tetradecanoate;
(R)-2-(2-amino-3-(4-((6-amino-2-(butylamino)-8-hydroxy-9H-purin-9-yl)methyl)phenylamino)-3-oxopropylthio)ethyl palmitate;
(R)-2-(2-amino-3-(4-((6-amino-2-(butylamino)-8-hydroxy-9H-purin-9-yl)methyl)phenylamino)-3-oxopropylthio)ethyl stearate;
2-((S)-2-amino-3-((S)-1-(4-((6-amino-2-(butylamino)-8-hydroxy-9H-purin-9-yl)methyl)phenylamino)-3-hydroxy-1-oxopropan-2-ylamino)-3-oxopropylthio)ethyl tetradecanoate;
2-((S)-2-amino-3-((S)-1-(4-((6-amino-2-(butylamino)-8-hydroxy-9H-purin-9-yl)methyl)phenylamino)-3-hydroxy-1-oxopropan-2-ylamino)-3-oxopropylthio)ethyl palmitate;
2-((S)-2-amino-3-((S)-1-(4-((6-amino-2-(butylamino)-8-hydroxy-9H-purin-9-yl)methyl)phenylamino)-3-hydroxy-1-oxopropan-2-ylamino)-3-oxopropylthio)ethyl stearate;
2-((R)-2-amino-3-((S)-5-(4-((6-amino-2-(butylamino)-8-hydroxy-9H-purin-9-yl)methyl)phenylamino)-1-methoxy-1,5-dioxopentan-2-ylamino)-3-oxopropylthio)ethyl tetradecanoate;
(R)-2-(2-amino-3-(3-(4-amino-2-(ethoxymethyl)-1H-imidazo[4,5-c]quinolin-1-yl)propyl amino)-3-oxopropylthio)ethyl stearate;
(R)-2-(2-amino-3-(3-(4-amino-2-((ethylamino)methyl)-1H-imidazo[4,5-c]quinolin-1-yl)propylamino)-3-oxopropylthio)ethyl stearate.

As one embodiment of A, for example, but not limited to, structures derived from N1-glycinyl [4-((6-amino-2-(butylamino)-8-hydroxy-9H-purin-9-yl)methyl)benzoyl]spermine (CL307; InvivoGen) (containing a spermine structure, which can also serve as a linker (L)), 2-(4-((6-amino-2-(butylamino)-8-hydroxy-9H-purin-9-yl)methyl)benzamido)acetic acid (CL264; InvivoGen), Imiquimod (CAS: 99011-02-6), R-848 (CAS: 144875-48-9), and the like can be mentioned.

As one embodiment of B, for example, but not limited to, structures derived from S-[2,3-bis(palmitoyloxy)-(2RS)-propyl]-[R]-cysteinyl-[S]-seryl-[S]-lysyl-[S]-lysyl-[S]-lysyl-[S]-lysine x 3 CF3COOH (Pam2CSK4; InvivoGen), N-Palmitoyl-S-[2,3-bis(palmitoyloxy)-(2RS)-propyl]-[R]-cysteinyl-[S]-seryl-[S]-lysyl-[S]-lysyl-[S]-lysyl-[S]-lysine (Pam3CSK4; InvivoGen), PamCSK4, Pam2Cys, Pam3Cys, PamCys, and the like can be mentioned.

As one embodiment of L, for example, but not limited to, a cationic linker or the like can be mentioned. Furthermore, as a specific embodiment of L, for example, the structure represented by the following formula (L-1) can be mentioned. 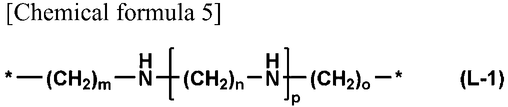 wherein,
m represents an integer from 0 to 4,
n represents an integer from 0 to 4,
o represents an integer from 0 to 4,
p represents an integer from 1 to 6, and
* indicates a bond to -NH-.

As one embodiment of L, for example, structures derived from spermine (m=3, n=4, o=3, p=1), putrescine (m=0, n=4, o=0, p=1), and spermidine (m=0, n=4, o=1, p=3) can be mentioned, with spermine being preferred, but the present invention is not limited thereto.

In another embodiment, the compound represented by formula (I) used as the adjuvant of the present invention may have a structure preferably used as L, such as a structure derived from spermine, not as a linker connecting A and B, but as a part of A or B, or as a non-active part other than A and B.

As a preferred embodiment of the compound represented by the general formula (I), the compound shown below ((20R)-20-amino-1-(4-((6-amino-2-(butylamino)-8-hydroxy-9H-purin-9-yl)methyl)phenyl)-1,4,19-trioxo-22-thia-2,5,9,14,18-pentaazapentacosane-24,25-diyl dipalmitate) can be mentioned. This compound is referred to as "TY001".

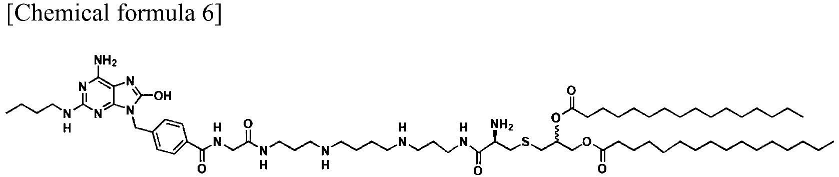

In addition, the R-isomer of TY001 ((20R,24R)-20-amino-1-(4-((6-amino-2-(butylamino)-8-hydroxy-9H-purin-9-yl)methyl)phenyl)-1,4,19-trioxo-22-thia-2,5,9,14,18-pentaazapentacosane-24,25-diyl dipalmitate) and the S-isomer ((20R,24S)-20-amino-1-(4-((6-amino-2-(butylamino)-8-hydroxy-9H-purin-9-yl)methyl)phenyl)-1,4,19-trioxo-22-thia-2,5,9,14,18-pentaazapentacosane-24,25-diyl dipalmitate) are novel compounds and are compounds of the present invention. That is, a further aspect of the present invention relates to the compound represented by the formula below, or a pharmaceutically acceptable salt thereof (hereinafter, these may be collectively referred to as "the compound of the present invention").

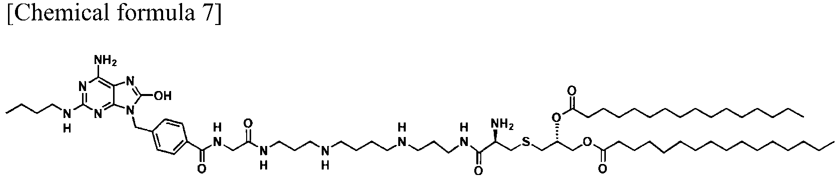

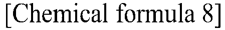

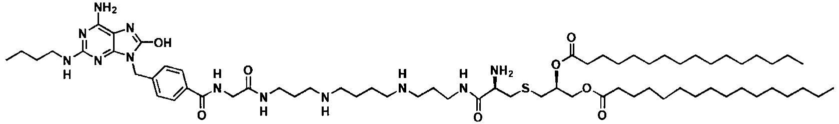

As used herein, "pharmaceutically acceptable salt" refers to a salt formed by bonding with an inorganic or organic base or acid, which is acceptable for administration into the body as a pharmaceutical. Such salts are described, for example, in Berge et al., J. Pharm. Sci. 66:1-19 (1977). When the compound of formula (I) has an acidic group such as a carboxylic acid group, examples of salts with such acidic groups include alkali metal salts and alkaline earth metal salts such as lithium, sodium, potassium, magnesium, and calcium; amine salts such as ammonia, methylamine, dimethylamine, trimethylamine, dicyclohexylamine, tris(hydroxymethyl)aminomethane, N,N-bis(hydroxyethyl)piperazine, 2-amino-2-methyl-1-propanol, ethanolamine, N-methylglucamine, and L-glucamine; or salts with basic amino acids such as lysine, δ-hydroxylysine, and arginine. When the compound of formula (I) has a basic group such as an amino group, examples of salts with such basic groups include salts with hydrochloric acid, hydrobromic acid, phosphoric acid, sulfuric acid, nitric acid, boric acid, etc. (inorganic acid salts); salts with methanesulfonic acid, benzenesulfonic acid, p-toluenesulfonic acid, formic acid, propionic acid, acetic acid, trifluoroacetic acid, lactic acid, fumaric acid, malic acid, oxalic acid, benzoic acid, mandelic acid, cinnamic acid, maleic acid, tartaric acid, citric acid, succinic acid, malonic acid, tosylic acid, glycolic acid, glucuronic acid, ascorbic acid, nicotinic acid, salicylic acid, etc. (organic acid salts); or salts with acidic amino acids such as aspartic acid and glutamic acid. The preparation of these salts can be carried out by conventional means. It should be noted that the above examples should not be used to limit the interpretation of "pharmaceutically acceptable salt." That is, "pharmaceutically acceptable salt" should be interpreted broadly and is a term encompassing various types of salts. Unless otherwise explicitly stated, and unless clearly inappropriate, the compounds described herein also include hydrates or solvates of the compounds or their salts.

The adjuvant preparation of the present invention and the vaccine of the present invention may contain one type of the adjuvant of the present invention, or may contain two or more types in combination. Furthermore, the adjuvant preparation of the present invention and the vaccine of the present invention may contain adjuvant components other than the adjuvant of the present invention.

The adjuvant of the present invention may be a commercially available product if it is available as a known compound, or it may be produced or purified by known production or purification methods. For example, it can be produced based on the methods described in the Examples below.

Induction of mucosal immunity using the adjuvant of the present invention (or administration of the adjuvant of the present invention) is typically performed by coadministering a vaccine composition containing the adjuvant of the present invention along with an antigen to a subject. However, if necessary, it may be administered separately from the antigen. For example, the adjuvant of the present invention or an adjuvant-containing formulation (not containing an antigen; hereinafter, this may be referred to as "the adjuvant formulation of the present invention") may be mucosally administered at an immunologically inducible timing before or after antigen administration. In this case, the adjuvant and the antigen may be administered via separate administration routes. When the adjuvant and antigen are administered via separate administration routes, conventional administration methods for the antigen include intradermal administration, intramuscular administration, and the like. Furthermore, the adjuvant formulation of the present invention can also be administered for purposes such as activation of innate immunity or suppression of allergies. The adjuvant of the present invention, as a vaccine composition containing an antigen, or as a composition not containing an antigen (adjuvant formulation), can typically be formulated into liquid preparations (suspensions or solutions, etc.), spray preparations (suspensions or solutions, etc.), powder preparations (semi-solid preparations or solid preparations, etc.), solid preparations, semi-solid preparations (pastes, creams), film preparations, sheet preparations, or gel preparations (preferably in a form that dissolves by bodily fluids and/or body temperature). The adjuvant preparation of the present invention and the vaccine composition of the present invention are administered, for example, by dropping, spraying, coating, or applying into the nasal cavity, oral cavity, or other mucous membranes.

Examples of liquid preparations and spray preparations (solutions) include those in which the adjuvant and/or antigen are dissolved in purified water, buffer solutions, and the like.

Examples of liquid preparations and spray preparations (suspensions) include those in which the adjuvant and/or antigen are suspended in purified water, buffer solutions, etc., along with methylcellulose, hydroxymethylcellulose, polyvinylpyrrolidone (PVP), gelatin, casein, and the like.

Examples of solid preparations and semi-solid preparations include those in which the adjuvant and/or antigen are mixed with oil/water type bases such as hydrophilic ointments and vanishing creams; or water/oil type bases such as hydrophilic petrolatum, purified lanolin, Aquaphor, Eucerin, Neocerin, hydrated lanolin, cold cream, and hydrophilic Plastibase, which have been stirred with oily solvents or water.

Examples of powder preparations include those in which the adjuvant and/or antigen are thoroughly mixed with methylcellulose, hydroxymethylcellulose, hydroxypropylmethylcellulose, and the like.

Examples of film preparations and sheet preparations include molded articles having a laminated structure including a mucoadhesive layer, which contains water-soluble matrix materials (e.g., pullulan, polyvinyl alcohol (PVA), hydroxypropylcellulose (HPC), polyvinylpyrrolidone (PVP), gelatin, starch, etc.).

Commonly used absorption enhancers, surfactants, preservatives, stabilizers, humectants, moisturizers, solubilizers, and the like can be added to these formulations as needed. Known formulation methods can be used for the production of the vaccine composition and adjuvant preparation of the present invention.

It is preferred that the vaccine composition and the adjuvant preparation of the present invention further contain chondroitin sulfate and/or a salt thereof. This can further enhance the antibody-inducing effect. As the salt of chondroitin sulfate, an alkali metal salt of chondroitin sulfate is preferred, and sodium chondroitin sulfate is more preferred. The amount of chondroitin sulfate and/or its salt to be added can be appropriately adjusted depending on the type of antigen, etc., but it is preferably included in an amount of about 0.001 to 10 mass% or about 0.008 to 3 mass% in the adjuvant preparation containing the compound represented by formula (I) or a pharmaceutically acceptable salt thereof. Furthermore, the amount of chondroitin sulfate and/or its salt to be added is preferably included in an amount of about 0.001 to 10 mass% or about 0.008 to 3 mass% in the vaccine preparation containing the compound represented by formula (I) or a pharmaceutically acceptable salt thereof and an antigen.

The content of the compound represented by formula (I) or a pharmaceutically acceptable salt thereof in the vaccine of the present invention and the adjuvant preparation of the present invention is not particularly limited as long as it is an amount sufficient to exert the immune-inducing effect of the present invention. The content of the compound represented by formula (I) or a pharmaceutically acceptable salt thereof relative to the entire formulation may be 0.00001 to 100 mass%, 0.0001 to 90 mass%, 0.001 to 50 mass%, 0.01 to 10 mass%, or 0.1 to 2.5 mass%. Such a content may vary depending on the type of adjuvant, etc., but can be conventionally determined by those skilled in the art.

The dosage of the adjuvant of the present invention can be appropriately determined depending on the antigen, type of adjuvant, subject of administration, administration method, administration form, etc. For example, taking an adult weighing 60 kg as an example, in the case of parenteral administration, the daily dose of the compound represented by formula (I) or a pharmaceutically acceptable salt thereof may be about 0.0001 mg to about 5 g, about 0.001 to about 500 mg, about 0.01 to about 50 mg, or about 0.1 to about 5 mg. When administered to other animals, the above dose is converted to a dose per unit body weight, and then multiplied by the body weight of the animal to be administered to obtain the appropriate dose.

A further aspect of the present invention relates to a vaccine composition (hereinafter, which may be referred to as "the vaccine composition of the present invention" or "the vaccine of the present invention") comprising at least one type of antigen and the adjuvant of the present invention. The vaccine of the present invention is typically a mucosal vaccine capable of inducing IgA antibodies against the antigen contained in the vaccine in the mucous membrane upon mucosal administration. One aspect of the present invention is a vaccine for inducing mucosal immunity. Another aspect of the present invention is a vaccine for mucosal administration.

The content of the antigen in the vaccine composition of the present invention is not particularly limited as long as it is an amount sufficient to exert the immune-inducing effect of the present invention. The content of the antigen relative to the entire composition may be 0.00001 to 70 mass%, 0.0001 to 60 mass%, 0.001 to 50 mass%, 0.01 to 30 mass%, 0.05 to 10 mass%, 0.1 to 5 mass%, or 1 to 2.5 mass%. Such a content may vary depending on the type of antigen, etc., but can be conventionally determined by those skilled in the art.

The mass ratio of the antigen to the adjuvant contained in the vaccine composition of the present invention is not limited, but a ratio of 500:1 to 1:50 is preferable.

The dose of the vaccine composition of the present invention can be appropriately determined depending on the antigen, the type of adjuvant, the subject, the administration method, the dosage form, etc. For example, for an adult weighing 60 kg, the daily dose of antigen may be approximately 0.0001 to approximately 1000 mg, approximately 0.001 to approximately 500 mg, approximately 0.01 to approximately 100 mg, approximately 0.1 to approximately 50 mg, or approximately 1 to approximately 10 mg. When administering to other animals, the above dose is converted to a dose per unit body weight and then multiplied by the body weight of the animal to be administered.

The timing of administration is not particularly limited as long as a therapeutic or prophylactic effect can be exerted, but in the case of a prophylactic vaccine, it is preferable to administer it at least two weeks before the expected time of infection.

The subjects for administration can be appropriately determined depending on the type of antigen contained in the vaccine composition, and examples include humans and mammals other than humans. Examples of mammals other than humans include mice, rats, hamsters, guinea pigs, rabbits, pigs, cows, goats, horses, sheep, dogs, cats, monkeys, orangutans, and chimpanzees.

Diseases targeted by infectious disease vaccines include infectious diseases caused by pathogenic viruses, pathogenic bacteria, pathogenic fungi, or parasites from which the antigen is derived. Diseases or conditions targeted by non-infectious disease vaccines are not particularly limited, but include Alzheimer's disease, Parkinson's disease, Creutzfeldt-Jakob disease, arteriosclerosis, hypertension, multiple sclerosis, type 1 diabetes, myasthenia gravis, allergies such as hay fever, bronchial asthma, cancer, poisoning by nicotine, cocaine, phencyclidine, methamphetamine, heroin, morphine, etc., rheumatoid arthritis, contraception, obesity, and osteoporosis.

Furthermore, the adjuvant preparations and vaccine compositions of the present invention can induce humoral immunity, as shown in the examples below, and thus can be used for inducing humoral immunity.

Moreover, the adjuvant preparations and vaccine compositions of the present invention can induce the production of IgA antibodies in the mucosa, as shown in the examples below, and thus can be used for inducing the production of IgA antibodies in the mucosa.

Moreover, the adjuvant preparations and vaccine compositions of the present invention can induce the production of IgG antibodies in the blood in addition to the production of IgA antibodies in the mucosa, as shown in the examples below, and thus can be used for inducing the production of IgG antibodies in the blood.

### Examples

Hereinafter, the present invention will be described in more detail with reference to examples, but the present invention is not limited to these examples as long as it does not exceed the gist thereof.

### <Synthesis of Compounds>

Compounds were synthesized as follows.

### (General Procedure)

¹H NMR spectra were recorded on a Bruker AVANCE III (300 MHz). Chemical shifts are reported in parts per million (ppm) downfield from tetramethylsilane (δ) as an internal standard in deuterated solvent, and coupling constants (J) are reported in hertz (Hz). Data are reported as: chemical shift, integration, multiplicity (s = singlet, d = doublet, t = triplet, q = quartet, m = multiplet, dd = doublet of doublets, brs = broad singlet) and coupling constant. All solvents and reagents were commercially available. The optical purity of compounds was determined by UFLC (Shimadzu Corporation) analysis.

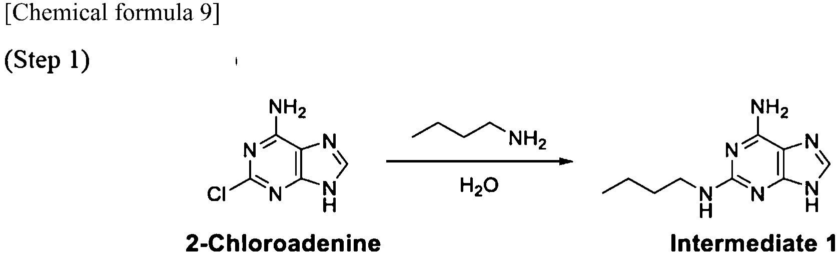

### (Step 1)

A mixture consisting of 2-chloroadenine (10.0 g, 59 mmol), butylamine (43 g, 589 mmol), and water (60 mL) was reacted in an autoclave at 180°C for 10 hours. After cooling, the reaction solution was concentrated under reduced pressure, and water was poured into the residue to precipitate a solid. The obtained precipitate was collected by filtration, washed with water and isopropyl ether (IPE) to obtain Intermediate 1 (10.2 g, 49.5 mmol, 83.9%) as a green solid.

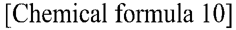

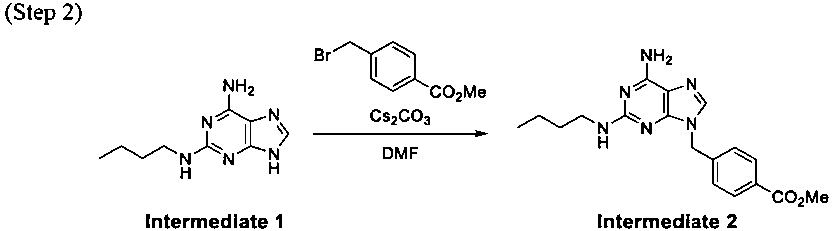

### (Step 2)

To a suspension of Intermediate 1 (3.50 g, 17.0 mmol) and cesium carbonate (Cs₂CO₃) (6.12 g, 18.7 mmol) in N,N-dimethylformamide (DMF) (70 mL) was added methyl 4-(bromomethyl)benzoate (5.05 g, 22.1 mmol) dropwise at room temperature, and the resulting mixture was stirred overnight at room temperature. The reaction mixture was poured into ice water and extracted with ethyl acetate (EtOAc). The organic layer was separated, washed with water and brine, dried over magnesium sulfate (MgSO₄), and concentrated in vacuo. The residue was purified by column chromatography (silica gel, eluted with 30-100% ethyl acetate (EtOAc) in n-hexane, then 7% methanol (MeOH) in ethyl acetate (EtOAc)) to obtain Intermediate 2 (3.40 g, 9.59 mmol, 56.5%) as an off-white crystal.

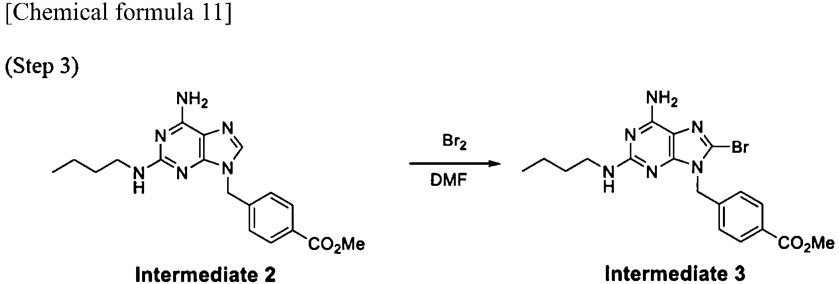

### (Step 3)

To a suspension of Intermediate 2 (5.00 g, 14.1 mmol) in N,N-dimethylformamide (DMF) (50 mL) was added bromine (Br2) (0.94 mL, 18.3 mmol) dropwise at room temperature, and the resulting mixture was stirred at room temperature for 2 hours. To the mixture were added 10% aqueous sodium thiosulfate (Na₂S₂O₃) solution (10 mL), water (100 mL), and triethylamine (Et3N) (10 mL), and the resulting mixture was stirred at room temperature for 1 hour. The obtained precipitate was collected by filtration and washed with water to obtain Intermediate 3 (5.10 g, 11.8 mmol, 83.4%) as a yellow solid.

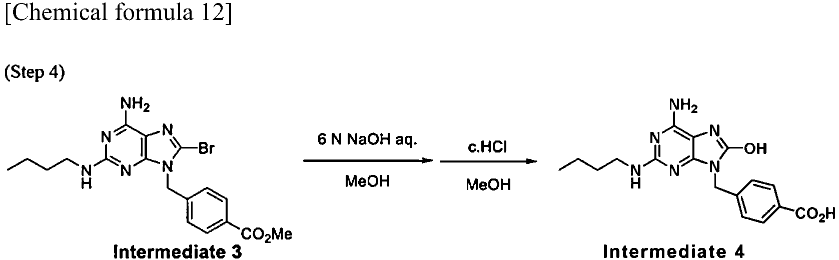

### (Step 4)

To a suspension of Intermediate 3 (2.50 g, 5.77 mmol) in methanol (MeOH) (25 mL) was added 6N aqueous sodium hydroxide (NaOH) solution (14.4 mL), and the resulting mixture was refluxed overnight. After cooling, 12N aqueous hydrochloric acid (HCl) solution (30.1 g) was added to the mixture, and the resulting mixture was stirred at 90°C for 3 days. After cooling, the pH was adjusted to 5 with 2N aqueous sodium hydroxide (NaOH) solution, and a solid was precipitated. The obtained precipitate was collected by filtration, washed with water, and dried under reduced pressure to obtain Intermediate 4 (1.90 g, 5.33 mmol, 92.4%) as a pale yellow solid.

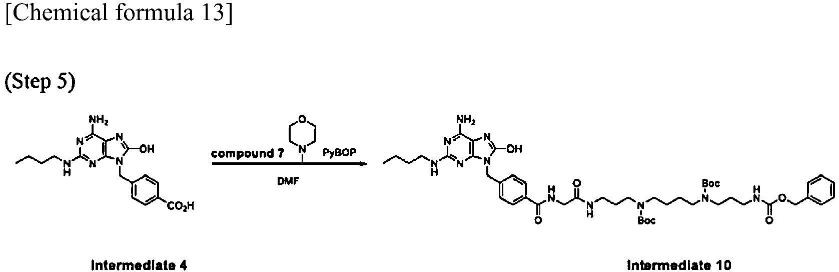

### (Step 5)

To a suspension of Intermediate 4 (720 mg, 2.02 mmol) in dry N,N-dimethylformamide (DMF) (20 mL) were added compound 7 (1.32 g, 2.22 mmol), PyBOP (registered trademark) (hexafluorophosphonium tripyrrolidinophosphonium) (1.58 g, 3.03 mmol), and N-methylmorpholine (1.1 g, 10.1 mmol), and the resulting mixture was stirred overnight at room temperature. The mixture was poured into water and extracted with ethyl acetate (EtOAc) and tetrahydrofuran (THF). The organic layer was separated, washed with saturated aqueous sodium bicarbonate (NaHCO₃) solution, 0.1N aqueous hydrochloric acid (HCl) solution, water, and brine, dried over magnesium sulfate (MgSO₄), and concentrated in vacuo. The residue was purified by column chromatography (silica gel, eluted with 0-25% methanol (MeOH) in ethyl acetate (EtOAc)) to obtain Intermediate 10 (1.50 g, 1.61 mmol, 79.7%) as a pale yellow amorphous solid.

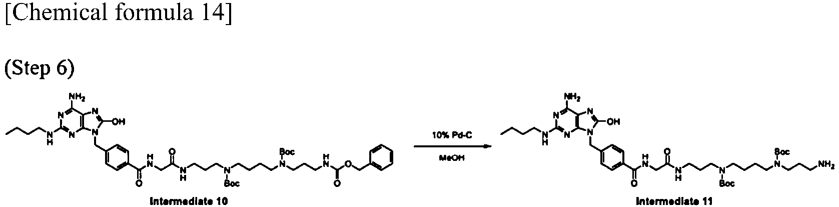

### (Step 6)

A mixture containing Intermediate 10 (1.50 g, 1.61 mmol) and 10% palladium on carbon (Pd/C) (PE type) (514 mg) in methanol (MeOH) (30 mL) was hydrogenated at room temperature under balloon pressure for 6 hours. The catalyst was removed by filtration, and the filtrate was concentrated in vacuo to obtain Intermediate 11 (1.20 g, 1.50 mmol, 93.4%) as a white amorphous solid.

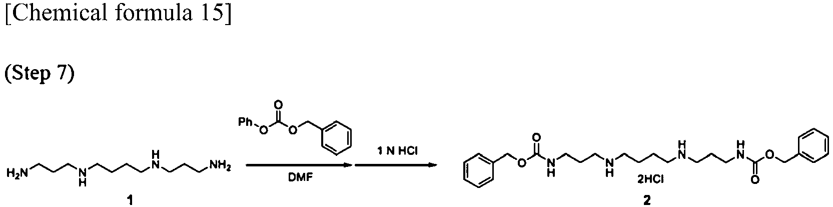

### (Step 7)

To a solution of Compound 1 (5.00 g, 24.7 mmol) in N,N-dimethylformamide (DMF) (50 mL) was added benzyl phenyl carbonate (10.2 mL, 51.9 mmol) at room temperature, and the resulting mixture was stirred overnight at room temperature. 1N aqueous hydrochloric acid (HCl) solution (55 mL) was added to the mixture, and the resulting mixture was stirred at room temperature for 1 hour. The obtained precipitate was collected by filtration and washed with ethyl acetate (EtOAc) to obtain Compound 2 (8.60 g, 16.0 mmol, 64.0%) as a white crystal.

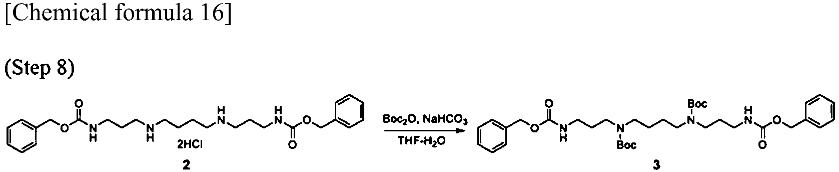

### (Step 8)

To a solution of Compound 2 (8.60 g, 15.8 mmol) and sodium bicarbonate (NaHCO₃) (1.33 g, 15.8 mmol) in water (27 mL) were added di-tert-butyl dicarbonate (Boc₂O) (11 mL, 47.5 mmol) and tetrahydrofuran (THF) (43 mL) at room temperature, and the resulting mixture was stirred at room temperature for 2 hours. The mixture was poured into water and extracted with ethyl acetate (EtOAc). The organic layer was separated, washed with brine, dried over magnesium sulfate (MgSO₄), and concentrated in vacuo. The residue was purified by column chromatography (silica gel, eluted with 10-100% ethyl acetate (EtOAc) in n-hexane) to obtain Compound 3 (10.6 g, 15.8 mmol, quantitative) as a colorless oil.

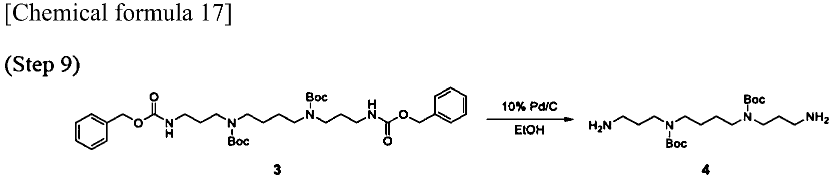

### (Step 9)

A mixture of Compound 3 (1.70 g, 2.53 mmol) and 10% palladium on carbon (Pd/C) (PE type) (539 mg) in ethanol (EtOH) (35 mL) was hydrogenated at room temperature under balloon pressure for 2 hours. The catalyst was removed by filtration, and the filtrate was concentrated in vacuo to obtain Compound 4 (1.02 g, 2.53 mmol, quantitative) as a colorless oil.

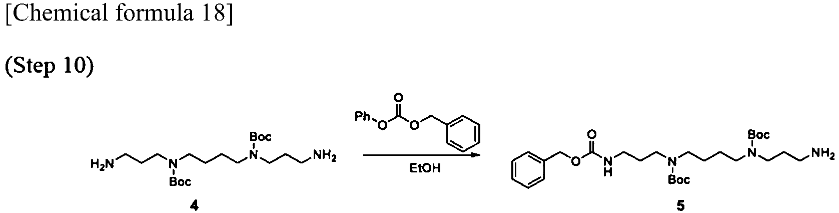

### (Step 10)

To a solution of Compound 4 (1.02 g, 2.53 mmol) in ethanol (EtOH) (20 mL) was added benzyl phenyl carbonate (0.50 mL, 2.53 mmol) at 0°C, and the resulting mixture was stirred overnight at room temperature. The solvent was evaporated in vacuo, and the residue was purified by column chromatography (NH silica gel, eluted with 50-100% ethyl acetate (EtOAc) in n-hexane) to obtain Compound 5 (0.60 g, 1.10 mmol, 44.0%) as a colorless oil.

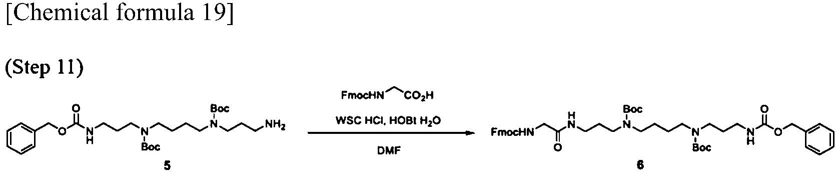

### (Step 11)

To a solution of (((9H-fluoren-9-yl)methoxy)carbonyl)glycine (499 mg, 1.68 mmol) in DMF (10 mL) were added N-ethyl-N'-(3-dimethylaminopropyl)carbodiimide hydrochloride (WSC·HCl) (321 mg, 1.68 mmol), triethylamine (Et₃N) (0.39 mL, 2.79 mmol), 1-hydroxybenzotriazole monohydrate (HOBt·H₂O) (257 mg, 1.68 mmol), and Compound 5 (600 mg, 1.12 mmol) at room temperature, and the resulting mixture was stirred overnight at room temperature. The mixture was poured into water and extracted with ethyl acetate (EtOAc). The organic layer was separated, washed with 0.1N aqueous hydrochloric acid (HCl) solution and brine, dried over magnesium sulfate (MgSO₄), and concentrated in vacuo. The residue was purified by column chromatography (silica gel, eluted with 50-100% ethyl acetate (EtOAc) in n-hexane) to obtain Compound 6 (710 mg, 0.87 mmol, 77.8%) as a colorless amorphous solid.

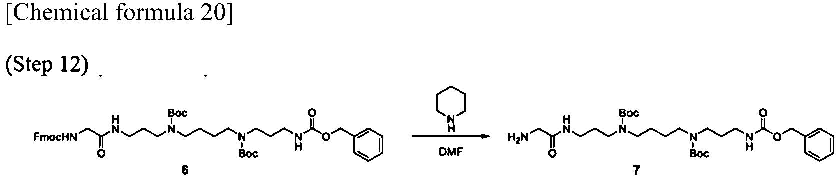

### (Step 12)

To a solution of Compound 6 (710 mg, 0.87 mmol) in N,N-dimethylformamide (DMF) (7 mL) was added piperidine (0.86 mL, 8.70 mmol) at room temperature, and the resulting mixture was stirred at room temperature for 1 hour. The mixture was poured into 1N aqueous sodium hydroxide (NaOH) solution and extracted with ethyl acetate (EtOAc). The organic layer was separated, washed with water and brine, dried over magnesium sulfate (MgSO₄), and concentrated in vacuo to obtain Compound 7.

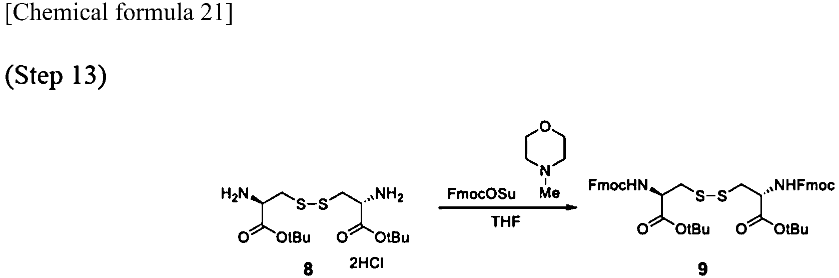

### (Step 13)

To a suspension of L-cystine bis(t-butyl ester) dihydrochloride ((H-Cys-OtBu)₂·2HCl) (Compound 8) (3.00 g, 7.05 mmol) and 9-fluorenylmethyl succinimidyl carbonate (3.81 g, 11.3 mmol) in tetrahydrofuran (THF) (30 mL) was added 4-methylmorpholine (3.1 mL, 28.2 mmol) at 0°C, and the resulting mixture was stirred at 0°C for 3 hours, then overnight at room temperature. After evaporation of the solvent, the residue was dissolved in ethyl acetate (EtOAc), washed with 0.1N aqueous hydrochloric acid (HCl) solution, water, and brine. The organic layer was separated, dried over magnesium sulfate (MgSO₄), and concentrated in vacuo. The residue was purified by column chromatography (silica gel, eluted with 10-40% ethyl acetate (EtOAc) in n-hexane) to obtain Compound 9 (3.50 g, 4.39 mmol, 62.3%) as a white crystal.

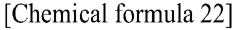

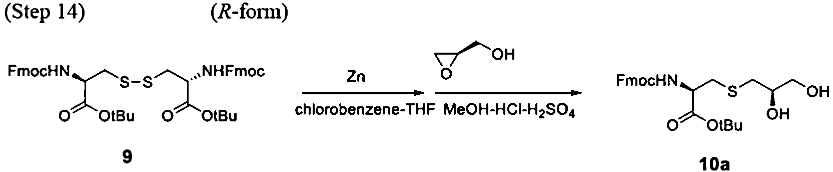

### (Step 14: R-form)

To a solution of Compound 9 (1.25 g, 1.57 mmol) in chlorobenzene (10 mL) and tetrahydrofuran (THF) (20 mL) were added zinc powder (718 mg, 11.0 mmol) and a freshly prepared mixture of methanol (MeOH), conc. hydrochloric acid (c.HCl), and sulfuric acid (H₂SO₄) (100:7:1, 4 mL) at 0°C, and the resulting mixture was stirred at 0°C for 30 minutes. To the mixture was added (R)-oxiran-2-ylmethanol (697 mg, 9.41 mmol). The resulting mixture was stirred at 40°C for 2 hours. The mixture was poured into water and extracted with ethyl acetate (EtOAc). The organic layer was separated, washed with brine, dried over magnesium sulfate (MgSO₄), and concentrated in vacuo. The residue was purified by column chromatography (silica gel, eluted with 20-100% ethyl acetate (EtOAc) in n-hexane) to obtain Compound 10a (1.21 g, 2.56 mmol, 81.5%) as a colorless amorphous solid.

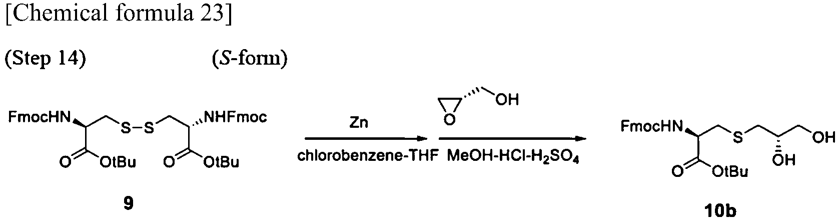

### (Step 14: S-form)

To a solution of Compound 9 (1.25 g, 1.57 mmol) in chlorobenzene (10 mL) and tetrahydrofuran (THF) (20 mL) were added zinc powder (718 mg, 11.0 mmol) and a freshly prepared mixture of methanol (MeOH), conc. hydrochloric acid (c.HCl), and sulfuric acid (H₂SO₄) (100:7:1, 4 mL) at 0°C, and the resulting mixture was stirred at 0°C for 30 minutes. To the mixture was added (S)-oxiran-2-ylmethanol (697 mg, 9.41 mmol). The resulting mixture was stirred at 40°C for 2 hours. The mixture was poured into water and extracted with ethyl acetate (EtOAc). The organic layer was separated, washed with brine, dried over magnesium sulfate (MgSO₄), and concentrated in vacuo. The residue was purified by column chromatography (silica gel, eluted with 20-100% ethyl acetate (EtOAc) in n-hexane) to obtain Compound 10b (1.20 g, 2.53 mmol, 84.1%) as a colorless amorphous solid.

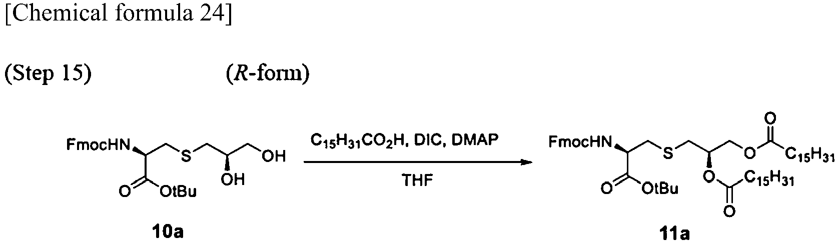

### (Step 15: R-form)

To a solution of Compound 10a (1.00 g, 2.11 mmol) and palmitic acid (1.62 g, 6.33 mmol) in tetrahydrofuran (THF) (20 mL) were added N,N'-diisopropylcarbodiimide (DIC) (0.98 mL, 6.33 mmol) and 4-dimethylaminopyridine (DMAP) (103 mg, 0.85 mmol) at room temperature, and the resulting mixture was stirred overnight at room temperature. The mixture was poured into water and extracted with ethyl acetate (EtOAc). The organic layer was separated, washed with 10% aqueous citric acid solution and brine, dried over magnesium sulfate (MgSO₄), and concentrated in vacuo. The residue was purified by column chromatography (silica gel, eluted with 0-30% ethyl acetate (EtOAc) in n-hexane) to obtain Compound 11a (2.01 g, 2.11 mmol, quantitative) as a white solid.

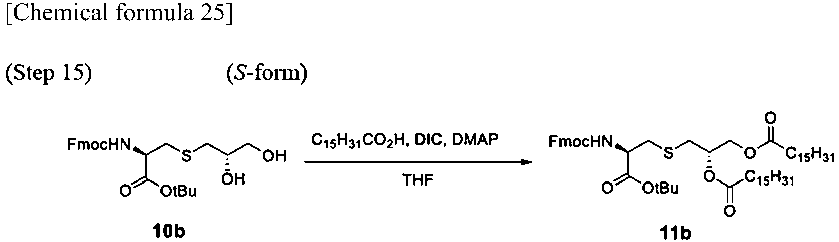

### (Step 15: S-form)

To a solution containing Compound 10b (1.00 g, 2.11 mmol) and palmitic acid (1.62 g, 6.33 mmol) in tetrahydrofuran (THF) (20 mL) were added N,N'-diisopropylcarbodiimide (DIC) (0.98 mL, 6.33 mmol) and 4-dimethylaminopyridine (DMAP) (103 mg, 0.85 mmol) at room temperature, and the resulting mixture was stirred overnight at room temperature. The mixture was poured into water and extracted with ethyl acetate (EtOAc). The organic layer was separated, washed with 10% aqueous citric acid solution and brine, dried over magnesium sulfate (MgSO₄), and concentrated in vacuo. The residue was purified by column chromatography (silica gel, eluted with 0% to 30% ethyl acetate (EtOAc) in n-hexane) to obtain Compound 11b (2.01 g, 2.11 mmol, quantitative) as a white solid.

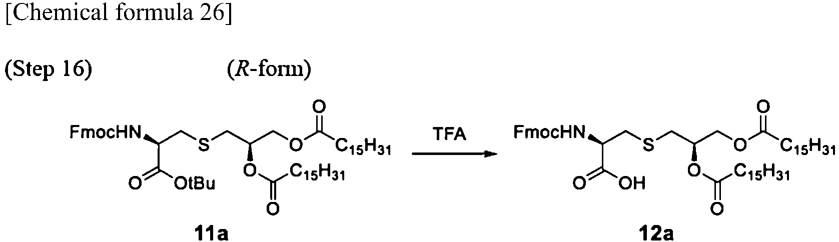

### (Step 16: R-form)

A mixture of Compound 11a (2.00 g, 2.10 mmol) and trifluoroacetic acid (TFA) (16.2 mL, 210 mmol) was stirred at room temperature for 1 hour. After evaporating trifluoroacetic acid, the residue was purified by column chromatography (silica gel, eluted with 10% to 100% ethyl acetate (EtOAc) in n-hexane) to obtain Compound 12a (1.60 g, 1.79 mmol, 85.0%) as a white solid.

### (Step 16: S-form)

A mixture of Compound 11b (2.01 g, 2.11 mmol) and trifluoroacetic acid (TFA) (16.2 mL, 211 mmol) was stirred at room temperature for 1 hour. After evaporating trifluoroacetic acid (TFA), the residue was purified by column chromatography (silica gel, eluted with 10% to 100% ethyl acetate (EtOAc) in n-hexane) to obtain Compound 12b (1.56 g, 1.74 mmol, 82.5%) as a white solid.

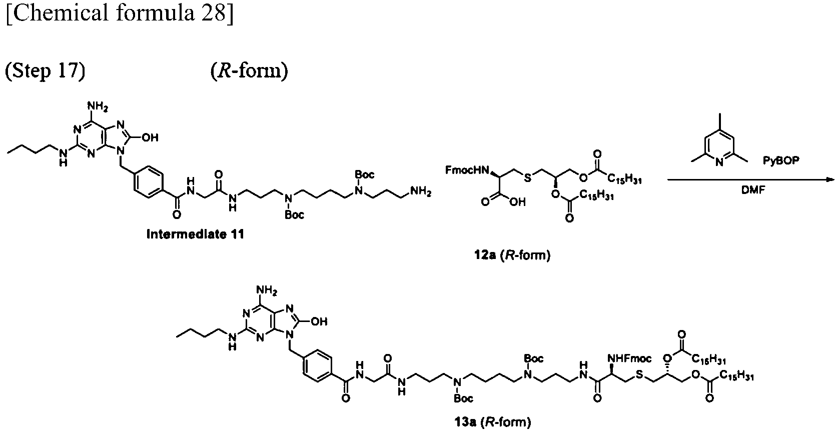

### (Step 17: R-form)

To a solution containing Intermediate 11 (1.34 g, 1.68 mmol) and Compound 12a (1.50 g, 1.68 mmol) in N,N-dimethylformamide (DMF) (30 mL) were added PyBOP (registered trademark) (tripyrrolidinophosphonium hexafluorophosphate) (1.75 g, 3.35 mmol) and 2,4,6-trimethylpyridine (0.44 mL, 3.35 mmol) at room temperature, and the resulting mixture was stirred for 1 hour at room temperature. The mixture was poured into water, extracted with ethyl acetate (EtOAc) and tetrahydrofuran (THF). The organic layer was separated, washed with 0.1N aqueous hydrochloric acid (HCl) solution, saturated aqueous sodium bicarbonate (NaHCO₃) solution, and brine, dried over magnesium sulfate (MgSO₄), and concentrated in vacuo. The residue was purified by column chromatography (silica gel, eluted with 0% to 20% ethyl acetate (EtOAc) in methanol (MeOH)) to obtain Compound 13a (2.40 g, 1.43 mmol, 85.5%) as a colorless amorphous solid.

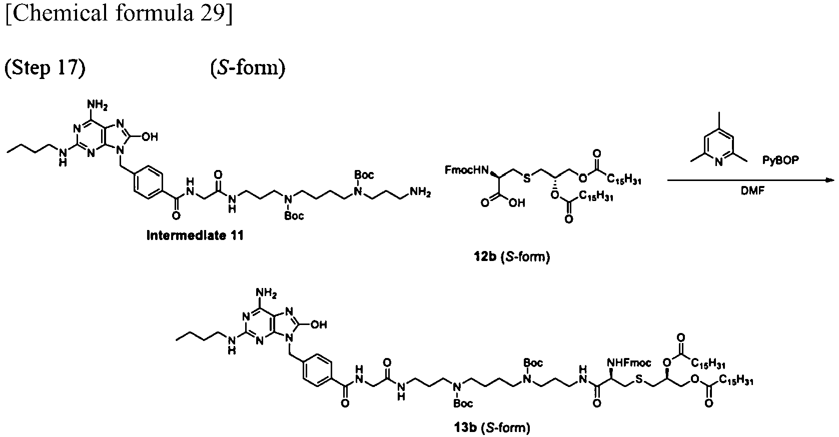

### (Step 17: S-form)

To a solution containing Intermediate 11 (1.34 g, 1.68 mmol) and Compound 12b (1.50 g, 1.68 mmol) in N,N-dimethylformamide (DMF) (30 mL) were added PyBOP (registered trademark) (tripyrrolidinophosphonium hexafluorophosphate) (1.75 g, 3.35 mmol) and 2,4,6-trimethylpyridine (0.44 mL, 3.35 mmol) at room temperature, and the resulting mixture was stirred for 1 hour at room temperature. The mixture was poured into water, extracted with ethyl acetate (EtOAc) and tetrahydrofuran (THF). The organic layer was separated, washed with 0.1N aqueous hydrochloric acid (HCl) solution, saturated aqueous sodium bicarbonate (NaHCO₃) solution, and brine, dried over magnesium sulfate (MgSO₄), and concentrated in vacuo. The residue was purified by column chromatography (silica gel, eluted with 0% to 20% ethyl acetate (EtOAc) in methanol (MeOH)) to obtain Compound 13b (2.20 g, 1.31 mmol, 78.3%) as a colorless amorphous solid.

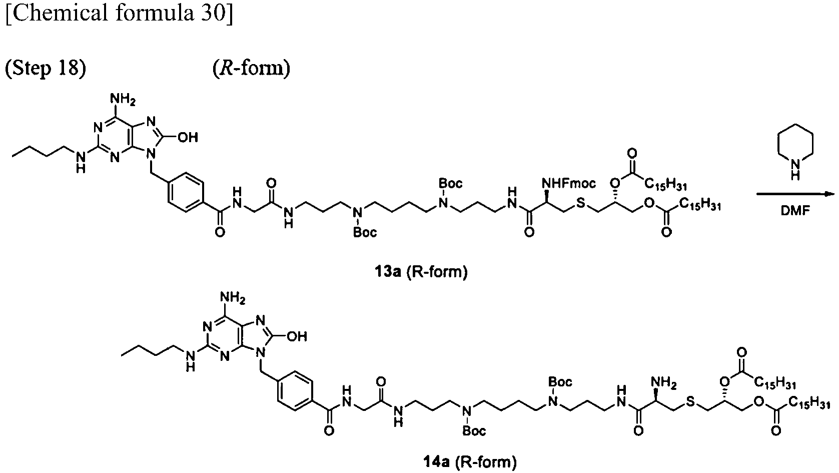

### (Step 18: R-form)

To a solution containing Compound 13a (2.40 g, 1.43 mmol) in N,N-dimethylformamide (DMF) (25 mL) was added piperidine (1.42 mL, 14.3 mmol) at room temperature, and the resulting mixture was stirred for 1 hour at room temperature. The mixture was poured into water, extracted with ethyl acetate (EtOAc) and tetrahydrofuran (THF). The organic layer was separated, washed with water and brine, dried over magnesium sulfate (MgSO₄), and concentrated in vacuo. The residue was purified by column chromatography (silica gel, eluted with 0% to 25% ethyl acetate (EtOAc) in methanol (MeOH)) to obtain Compound 14a (1.90 g, 1.31 mmol, 91.3%) as a colorless amorphous solid.

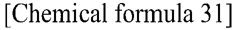

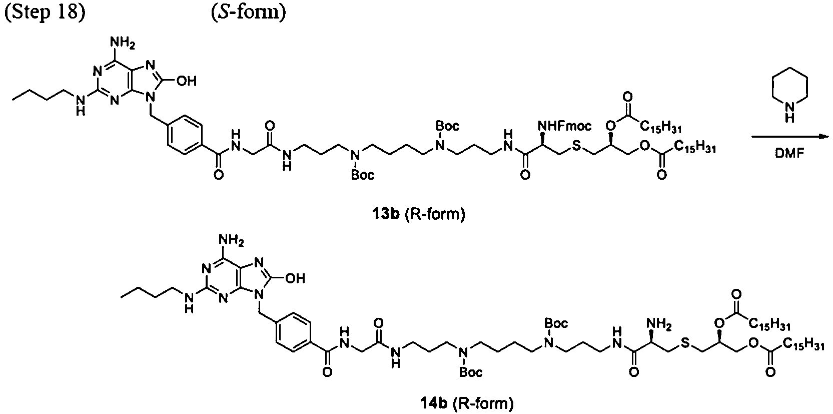

### (Step 18: S-form)

To a solution containing Compound 13b (2.20 g, 1.31 mmol) in N,N-dimethylformamide (DMF) (25 mL) was added piperidine (1.30 mL, 13.1 mmol) at room temperature, and the resulting mixture was stirred for 1 hour at room temperature. The mixture was poured into water, extracted with ethyl acetate (EtOAc) and tetrahydrofuran (THF). The organic layer was separated, washed with water and brine, dried over magnesium sulfate (MgSO₄), and concentrated in vacuo. The residue was purified by column chromatography (silica gel, eluted with 0% to 25% ethyl acetate (EtOAc) in methanol (MeOH)) to obtain Compound 14b (1.70 g, 1.17 mmol, 89.1%) as a colorless amorphous solid.

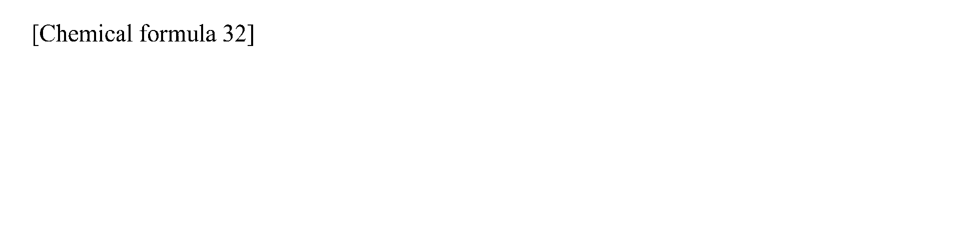

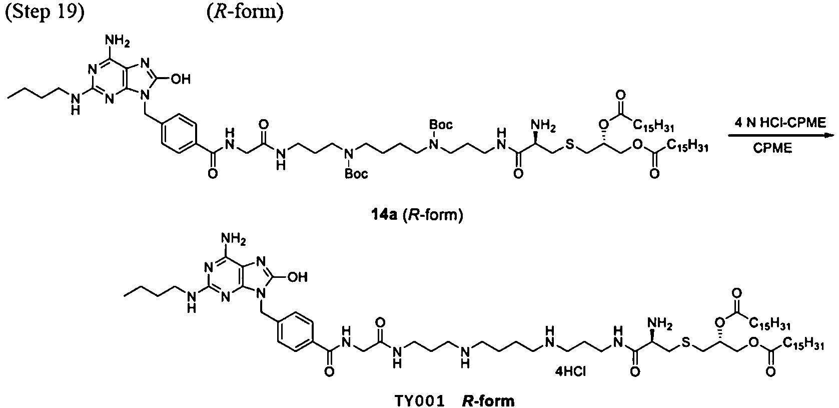

### (Step 19: R-form)

To a solution containing Compound 14a (1.90 g, 1.31 mmol) in cyclopentyl methyl ether (CPME) (10 mL) was added 4N hydrochloric acid (HCl) in cyclopentyl methyl ether (16.4 mL, 65.4 mmol) at room temperature, and the resulting mixture was stirred for 1 hour at room temperature. The solvent was evaporated under reduced pressure. Isopropyl ether (IPE) (50 mL) was added to the residue, and the resulting mixture was stirred for 30 minutes at room temperature. The resulting precipitate was collected by filtration, washed with isopropyl ether (IPE) to obtain TY001 (R-form) (1.78 g, 1.27 mmol, 97.3%) as a 4HCl salt in the form of a pale yellow amorphous solid. The NMR spectrum values of the synthesized compound are shown below.

¹H NMR (400 MHz, DMSO-d6, 298 K) δ 0.79-0.92 (9H, m), 1.14-1.39 (54H, m), 1.43-1.59 (7H, m), 1.64-1.75 (4H, m), 1.75-1.93 (4H, m), 2.22-2.34 (4H, m), 2.69-2.81 (1H, m), 2.82-3.06 (11H, m), 3.08-3.24 (3H, m), 3.25-3.38 (3H, m), 3.78-3.92 (2H, m), 3.94-4.06 (1H, m), 4.10-4.22 (1H, m), 4.26-4.38 (1H, m), 4.84-4.98 (2H, m), 5.07-5.22 (1H, m), 7.32-7.46 (2H, m), 7.82-7.90 (2H, m), 7.95-8.08 (1H, m), 8.12-8.18 (1H, m), 8.20-8.30 (2H, m), 8.41-8.56 (3H, m), 8.78-8.87 (1H, m), 8.89-9.02 (2H, m), 9.02-9.17 (3H, m), 11.00-11.18 (1H, m).

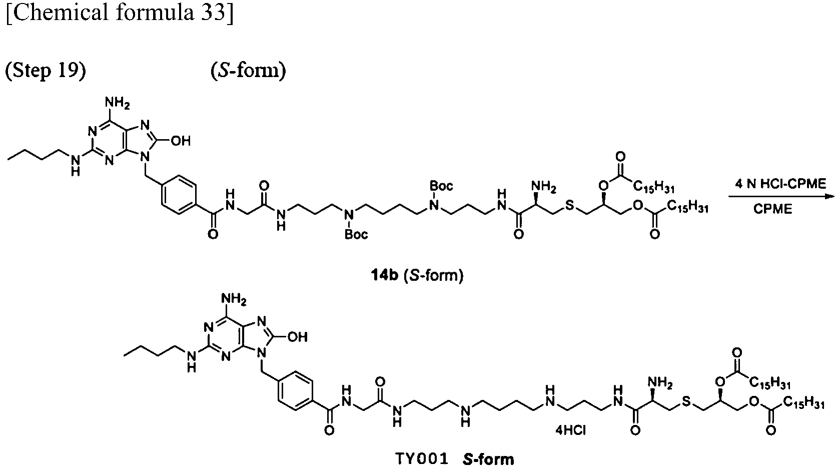

### (Step 19 S-form)

To a solution containing Compound 14b (1.70 g, 1.17 mmol) in cyclopentyl methyl ether (CPME) (10 mL) was added 4N hydrochloric acid (HCl) in cyclopentyl methyl ether (14.6 mL, 58.5 mmol) at room temperature, and the resulting mixture was stirred for 1 hour at room temperature. The solvent was evaporated under reduced pressure. Isopropyl ether (IPE) (50 mL) was added to the residue, and the resulting mixture was stirred for 30 minutes at room temperature. The resulting precipitate was collected by filtration, washed with isopropyl ether (IPE) to obtain TY001 (S-form) (1.53 g, 1.09 mmol, 93.5%) as a 4HCl salt in the form of a pale yellow amorphous solid. The NMR spectrum values of the synthesized compound are shown below.

¹H NMR (400 MHz, DMSO-d6, 298 K) δ0.80-0.90 (10H, m), 1.15-1.38 (62H, m), 1.42-1.56 (8H, m), 1.61-1.73 (5H, m), 1.73-1.87 (5H, m), 2.25-2.31 (4H, m), 2.70-2.81 (2H, m), 2.81-2.99 (14H, m), 3.11-3.23 (5H, m), 3.23-3.33 (3H, m), 3.80-3.87 (3H, m), 3.89-4.00 (2H, m), 4.10-4.16 (1H, m), 4.26-4.36 (1H, m), 4.85-4.97 (2H, m), 5.07-5.21 (1H, m), 7.34-7.44 (2H, m), 7.79-7.90 (2H, m), 8.06-8.19 (1H, m), 8.32-8.49 (3H, m), 8.72-8.84 (3H, m), 8.85-9.02 (4H, m), 10.52-10.89 (1H, m).

In the Examples described below, TY001 R-form 4HCl salt and TY001 S-form 4HCl salt were used as TY001 R-form and TY001 S-form, respectively.

### <Example 1: Confirmation of Nasal Adjuvant Effect of TY001 R-form and S-form>

### <<Infection Protection Ability Test by Nasal Administration of Influenza HA Antigen Vaccine in Combination with TY001 using mice>>

### (Method)

For the nasal administration test, 7-week-old female BALB/c mice were used, with 9 to 10 mice per group. Influenza virus HA protein antigen
(A/Singapore/GP1908/2015(IVR-180)(H1N1)pdm09) and influenza virus
(A/Singapore/GP1908/2015(IVR-180)(H1N1)pdm09) (Research Foundation for Microbial Diseases of Osaka University) were used. The vaccine groups included HA 0.1 µg alone, or in combination with 3 µg of TY001 R-form, or 3 µg of TY001 S-form as adjuvant.

The vaccine solution was administered nasally to mice at 5 µL per nostril (10 µL/mouse) twice, with a 3-week interval. Two weeks after the second administration (day 35), virus solution was administered at 2 µL per nostril (1x10^4 TCID50/mouse) to both nasal cavities. Three days later (day 38), serum and nasal lavage fluid were collected. Administration was performed under anesthesia by intraperitoneal injection of ketamine (10 mg/mL) + xylazine (1 mg/mL) at 0.2 mL/mouse.

### [Method for Collecting Serum and Nasal Lavage Fluid]

Three days after influenza virus inoculation, blood was collected under isoflurane anesthesia. After standing at 37°C for at least 1 hour and then at 4°C for at least 1 hour, the supernatant obtained by centrifugation was used as serum.

After euthanasia by whole blood collection, 1 mL of MEM medium containing 10% FBS was poured through a catheter inserted into the trachea, and the fluid discharged from the nose and mouth was collected and this procedure repeated 3 times. 1 mL of medium was added to the collected nasal lavage fluid, and the entire volume was centrifuged through a 0.22 µm filter (Ultrafree-CL-GV, 0.22 µm, Merck) to collect the filtrate.

### [Measurement of Viral Titer by TCID₅₀ method (Median tissue culture infectious dose, 50% infectious dose)]

MDCK cells (CCL-34, ATCC) were seeded into 96-well microplates (flat-bottom) (1x10⁴ cells/well) and cultured for 3 days (37°C, 5% CO₂) to prepare cell plates in a subconfluent state. Samples were prepared as 10^{0.5}-fold serial dilutions using MEM medium containing acetylated trypsin at a final concentration of 10 µg/mL. After washing the cell plates with PBS, 100 µL/well of the prepared samples were added and cultured at 37°C, 5% CO₂ for 3 days.

After culture, 10% formalin/PBS was added and left at room temperature for at least 10 min. After removing the liquid in the wells, NB staining solution was added and left at room temperature for at least 30 min. After washing the plate with tap water, it was dried at room temperature, 50 µL/well of 0.1N NaOH aqueous solution was added and stirred, and the absorbance at 630 nm was measured with a plate reader. The average OD₆₃₀ of the control cell cultured in MEM medium multiplied by 0.85 was set as the detection limit, and a positive result was determined when OD₆₃₀ was less than the detection limit. Based on this, the viral titer of the sample: TCID₅₀ was calculated by the Reed-Muench method.

### [ELISA Method]

HA antigen (A/Singapore/GP1908/2015(IVR-180)(H1N1)pdm09) was diluted and prepared at 1 µg/mL in 100 mM carbonate-bicarbonate buffer (pH 9.6) and added to 96-well plates. After overnight incubation at 4°C, plates were washed 3 times with 0.05% Tween 20 in PBS. 100 µL/well of 1% BSA-PBS was added and incubated at room temperature for 2 hours, then washed 3 times with 0.05% Tween 20 in PBS. Samples diluted (4-fold serial dilution) in 0.05% Tween 20 and 1% BSA in PBS were added at 50 µL/well and incubated overnight at 4°C. After washing with 0.05% Tween 20 in PBS, Horseradish Peroxidase-labeled goat anti-mouse IgA antibody (Bethyl) or anti-mouse IgG antibody (Bethyl) diluted in 0.05% Tween 20 and 1% BSA in PBS was added and incubated at room temperature for 2 hours. After washing 5 times with 0.05% Tween 20 in PBS, TMB chromogenic substrate solution was added, incubated in the dark at room temperature for 20 minutes, then the reaction was stopped by adding 2N sulfuric acid solution. Absorbance (450 nm) was measured immediately with a microplate reader.

### [HI Antibody Titer: Hemagglutination Inhibition Test]

To the serum sample, 3 volumes of RDE(II) "Seiken" (Denka Seiken) were added, incubated at 37°C for 18-20 hr, then heat-treated at 56°C for 30 min-1 hr. After cooling to room temperature, 6 volumes of PBS were added to the serum sample to prepare the test sample. For measurement, 1/10 volume of 50% chicken red blood cells (preserved chicken blood, Japan Bio Serum) was added to the test sample, incubated at room temperature for 1 hour, then centrifuged (1,200xg, 5 min) to obtain the supernatant. A 2-fold serial dilution was prepared, and A/Singapore/GP1908/2015(IVR-180)(H1N1)pdm09 (Denka Seiken) was added as the HI test antigen at 4HA/25µL and reacted at room temperature for 60 min. The reciprocal of the highest dilution factor of the sample showing complete hemagglutination inhibition was taken as the HI antibody titer. For HI antibody titers ≤ 10, the HI antibody titer was set to 5 for geometric mean calculation.

### (Results)

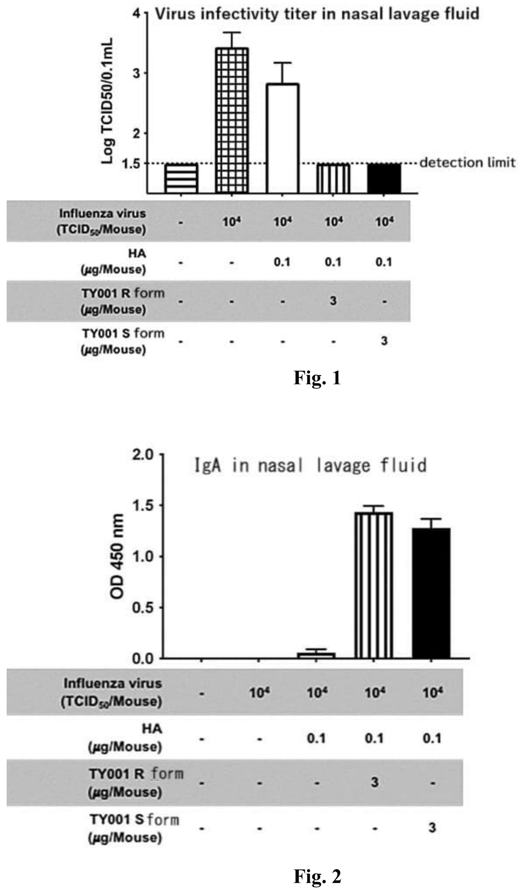

Fig. 1 shows the viral titer (mean + standard error) in the nasal lavage fluid. Nasal administration of the HA antigen vaccine alone did not show sufficient viral infection protective effect, but groups treated with HA antigen vaccine combined with TY001 R-form or S-form showed suppression of viral infection.

Fig. 2 shows the IgA level (mean + standard error) in the nasal lavage fluid. Nasal administration of the HA antigen vaccine alone did not induce antigen-specific IgA antibodies in the nasal cavity, but groups treated with HA antigen vaccine combined with TY001 R-form or S-form showed induction of antigen-specific IgA antibodies in the nasal cavity.

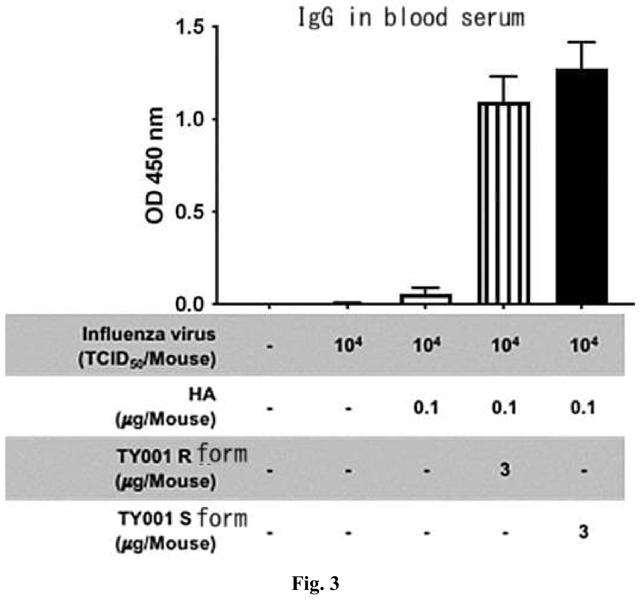

Furthermore, Fig. 3 shows the IgG level (mean + standard error) in serum, and Table 1 shows the HI antibody titer (geometric mean antibody titer) in serum. Nasal administration of the HA antigen vaccine alone did not show an increase in antigen-specific IgG antibodies or HI antibody titer in the blood, but groups treated with HA antigen vaccine combined with TY001 R-form or S-form showed an increase in antigen-specific IgG antibodies and HI antibody titer in serum.

**[Table 1]**

| **Table 1: Serum HI antibody titers when TY001 was used as a mucosal adjuvant** | | | | | |
|---|---|---|---|---|---|
| **HA antigen:** A/Singapore/GP1908/2015(IVR-180)(H1N1)pdm09 | | | | | |
| **Virus infection** | - | - | | | |
| **Antigen** | - | - | HA 0.1 *µ* g | | |
| **Adjuvant** | - | - | - | **TY001Rform3µg** | **TY001Sform3µg** |
| **Geometric Mean Titer (GMT)** | 16.2 | 15.2 | 18.7 | 69.6 | 85.7 |

### <Example 2: Confirmation of Adjuvant Effect by Nasal Administration of Compounds>

### <<Evaluation of IgA and IgG Antibody Induction Ability by Nasal Administration of Influenza HA Antigen Vaccine in Combination with CL401, CL413 or CL531>>

### (Method)

For the nasal administration test, 7-week-old female BALB/c mice were used, with 8 to 13 mice per group. Influenza HA protein antigen (A/California/7/2009(H1N1)pdm)
(Research Foundation for Microbial Diseases of Osaka University) was used.

Vaccine groups were set up with 1 µg of HA alone, and 1 µg of HA with CL401, CL413, or CL531 (InvivoGen) added as test compounds at 0.662 nmol or 6.62 nmol. The vaccine solution was administered nasally, 6.5 µL per nostril (13 µL/mouse), twice with a 2-week interval (day 0 and day 14). Plasma (day 27) and nasal lavage fluid (day 28) were collected 2 weeks after the boost immunization.

Antigen-specific IgA antibody levels in nasal lavage fluid and antigen-specific IgG antibody levels in plasma were measured by ELISA. Simultaneously, the antibody titers of the measured samples were expressed as relative values (unit) using a standard curve (1.2 - 0.005 unit) obtained by 2-fold serial dilution of stored mouse nasal lavage fluid or plasma as an internal standard.

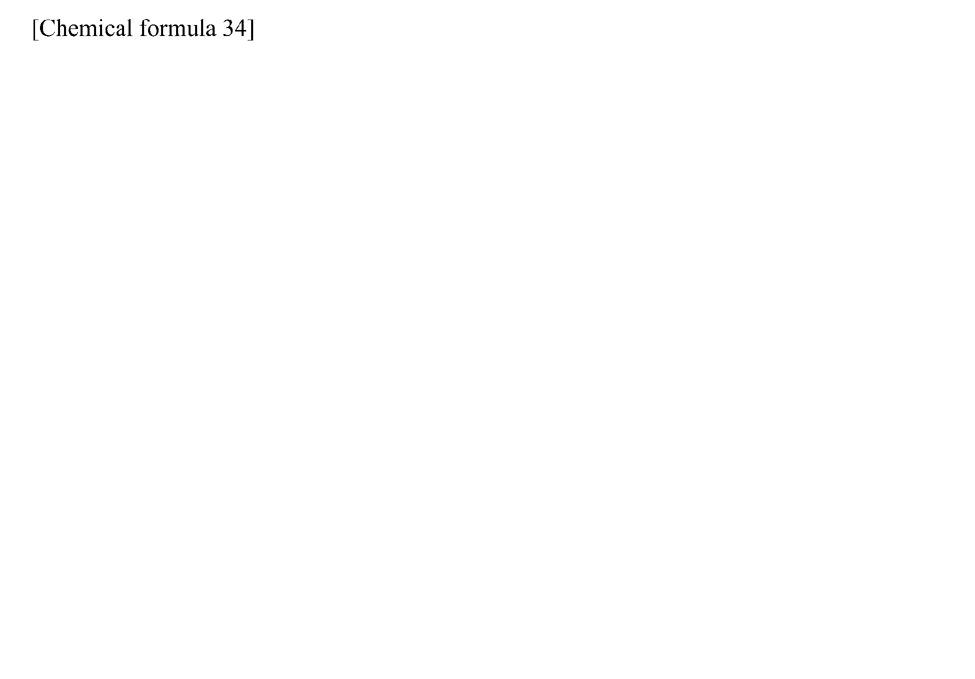

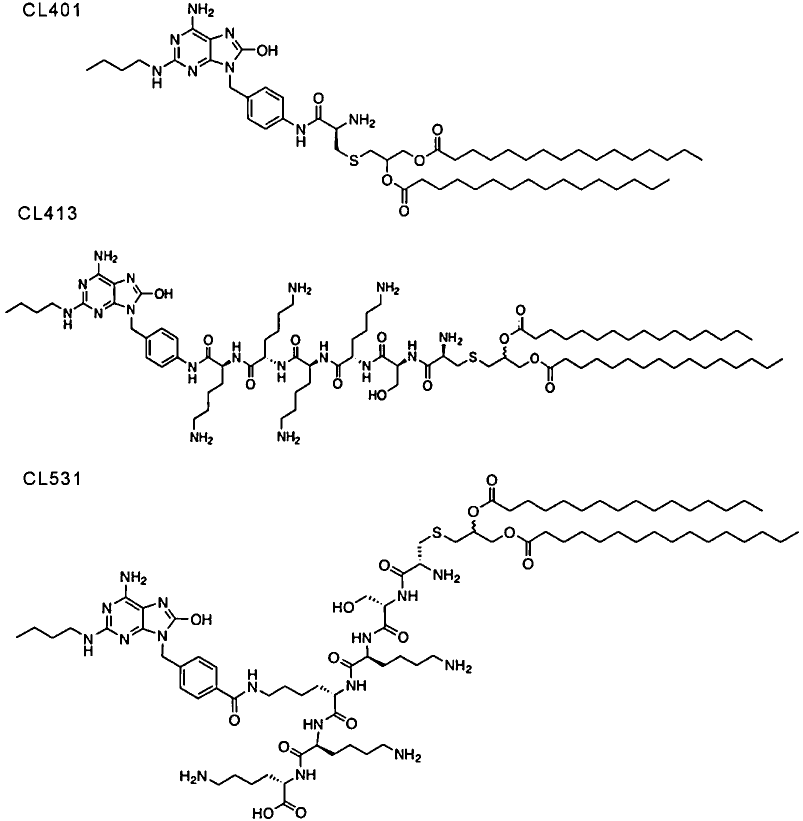

### (Results)

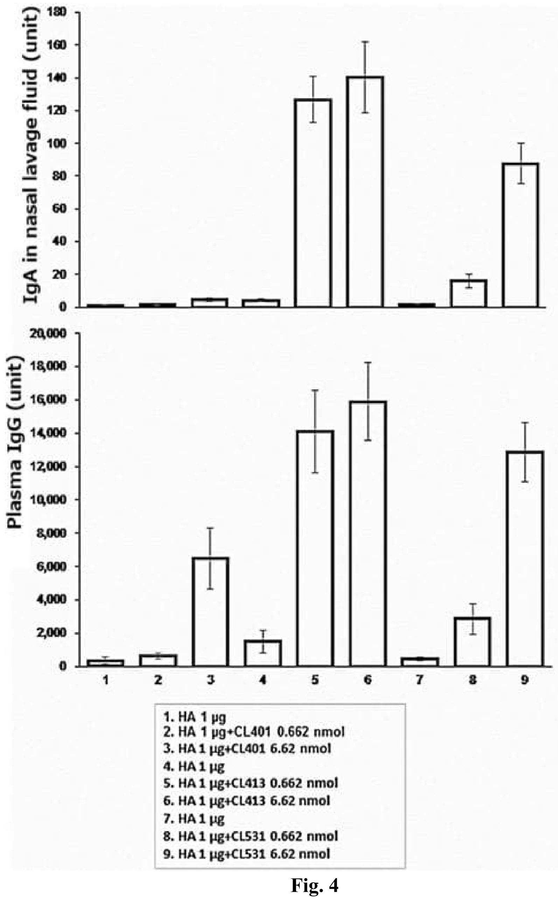

The results of three experiments conducted for each compound are summarized in Fig. 4 (upper panel: IgA antibody level in nasal lavage fluid, lower panel: IgG antibody level in plasma, mean ± standard error). Nasal administration of the HA antigen vaccine alone did not show sufficient induction of antigen-specific IgA in nasal lavage fluid or antigen-specific IgG antibodies in plasma. In groups treated with the test compounds, the induction of antigen-specific IgA and antigen-specific IgG antibodies in plasma was observed in the order of CL413 > CL531 > CL401.

### <Example 3: Confirmation of Adjuvant Effect by Nasal Administration of TY001 S-form using SARS-CoV-2 Antigen>

### <<Evaluation of Performance of TY001 S-form-Combined Nasal Vaccine with SARS-CoV-2 Spike Protein as Antigen in Mice>>

### (Method)

For the nasal administration test, 9-week-old female BALB/c mice were used, with 6 mice per group. SARS-CoV-2 S protein (ACROBiosystems) was used. Vaccine groups included 3 µg of S protein alone, and 3 µg of S protein with 0.3 µg of TY001 S-form as adjuvant.

The vaccine solution was administered intranasally to mice at 5 µL per nostril (10 µL/mouse) twice, with a 3-week interval (day 0 and 21). Serum and nasal lavage fluid were collected 2 weeks after the boost immunization (day 35). Administration was performed under anesthesia by intraperitoneal injection of ketamine (10 mg/mL) + xylazine (1 mg/mL) at 0.2 mL/mouse.

### [Method for Collecting Serum and Nasal Lavage Fluid]

Under isoflurane anesthesia, blood was collected from the inferior vena cava into a serum separating tube (Sansyo), and mixed by inversion. After standing at room temperature for 30 minutes, the supernatant obtained by centrifugation was used as serum.

After euthanasia by whole blood collection, 200 µL of MEM medium was flowed from the trachea, and the fluid discharged from the nose and mouth was collected. This procedure was performed twice. After centrifuging the collected solution at 4°C, 13,000 × g for 5 minutes, the supernatant was used as nasal lavage fluid.

### [ELISA Method]

A solution containing 1 µg/mL SARS-CoV-2 S protein trimer prepared in 100 mM carbonate-bicarbonate buffer (pH 9.6) was added at 50 µL/well to 96-well plates. After overnight incubation at 4°C, plates were washed with 0.05% Tween 20 in PBS. After incubation with 100 µL/well of 1% BSA-PBS at room temperature for 2 hours, plates were washed with 0.05% Tween 20 in PBS. Samples diluted (4-fold serial dilution) in 0.05% Tween 20 and 1% BSA in PBS were added at 50 µL/well and reacted overnight at 4°C. After washing with 0.05% Tween 20 in PBS, Horseradish Peroxidase-labeled goat anti-mouse IgA antibody (Bethyl) or anti-mouse IgG antibody (Bethyl) diluted 20,000-fold in 0.05% Tween 20 and 1% BSA in PBS was added at 50 µL/well and reacted at room temperature for 2 hours. After washing with 0.05% Tween 20 in PBS, 50 µL/well of TMB chromogenic substrate solution was added, incubated in the dark at room temperature for 20 minutes, then the reaction was stopped by adding 50 µL/well of Stop Solution for TMB Substrate (Abcam). Absorbance (OD450/630) was measured immediately with a microplate reader.

### (Results)

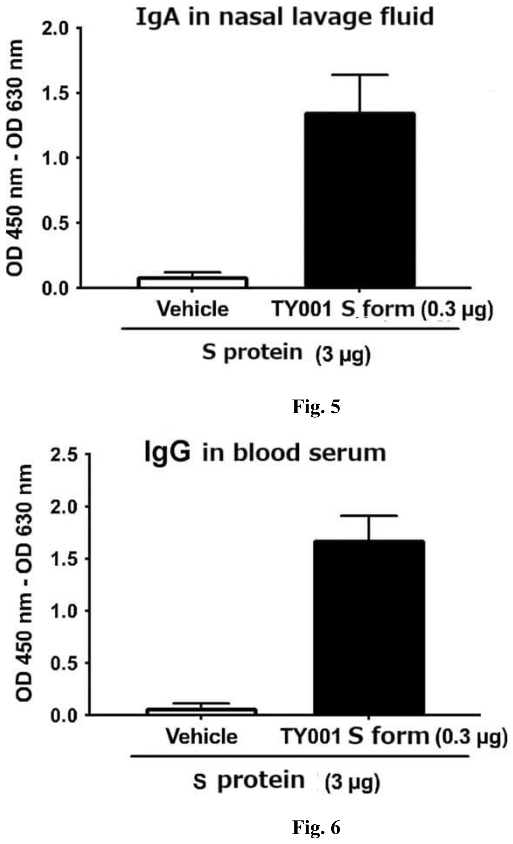

Fig. 5 shows the IgA level (mean + standard error) in the nasal lavage fluid. Nasal administration of the S protein antigen alone did not show sufficient induction of antigen-specific IgA antibodies, but groups treated with the S protein antigen combined with TY001 S-form showed induction of antigen-specific IgA antibodies in the nasal cavity.

Fig. 6 shows the IgG level (mean + standard error) in serum. Nasal administration of the S protein antigen alone did not show an increase in antigen-specific IgG antibody titer, but groups treated with the S protein antigen combined with TY001 S-form showed an increase in antigen-specific IgG antibodies.

### <Example 4: Evaluation of Nasal Adjuvant Effect of TY001 S-form>

### << Evaluation of Adjuvant Effect by Nasal Administration of Improved Formulation of Influenza HA Antigen Vaccine in Combination with TY001 S-form to Mice>>

### (Method)

For the nasal administration test, 7-week-old female BALB/c mice were used, with 8 mice per group. Influenza virus HA protein antigen A/Guangdong-Maonan/SWL1536/2019(CNIC-1909)(H1N1) (Research Foundation for Microbial Diseases of Osaka University) was used. Vaccine groups included HA 0.1 µg alone, or HA 0.1 µg with 0.1 µg of TY001 S-form as adjuvant, and groups with/without 0.01% chondroitin sulfate sodium as an additive.

The vaccine solution was administered intranasally to mice at 5 µL per nostril (10 µL/mouse) twice, with a 3-week interval. Two weeks after the second administration (day 35), nasal lavage fluid and serum were collected, and antigen-specific IgA levels in nasal lavage fluid and IgG levels in serum were measured by ELISA.

### (Results)

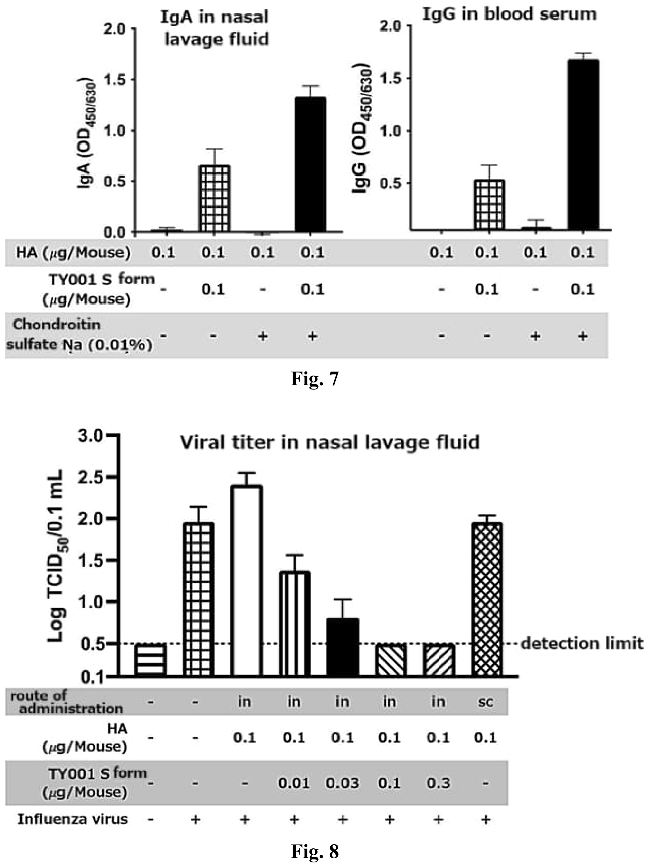

Fig. 7 shows the levels of IgA in nasal wash fluid and IgG in serum (mean + standard error). Co-administration of HA antigen vaccine with TY001 S-form induced antigen-specific IgA antibodies in the nasal cavity and IgG antibodies in the serum. Furthermore, the antibody-inducing effect for both IgA and IgG was stronger in the group that used a vaccine solution with TY001 S-form co-administered with 0.01% chondroitin sulfate sodium.

### <Example 5: Nasal Adjuvant Effect Evaluation Test of Improved TY001 S-form Formulation>

### <<Mouse Infection Protection Effect Test by Nasal Administration of Improved Influenza HA Antigen Vaccine Formulation Co-administered with TY001 S-form>>

### (Method)

For the nasal administration test, 7-week-old female BALB/c mice (9-10 mice per group) were used. Influenza virus HA protein antigen: A/Guangdong-Maonan/SWL1536/2019(CNIC-1909)(H1N1) and influenza virus: A/Guangdong-Maonan/SWL1536/2019(CNIC-1909)(H1N1) (Osaka University Microbial Disease Research Institute) were used. The vaccine formulations included HA 0.1 µg alone, and a formulation containing HA 0.1 µg co-administered with TY001 S-form at doses of 0.01-0.3 µg, and 0.01% chondroitin sulfate sodium as an additive. Vaccine solutions were administered into the nasal cavity, 5 µL into each nostril (10 µL/mouse), twice at a 3-week interval. As a control, a group subcutaneously administered with HA 0.1 µg alone was set up. Two weeks after the second administration (day 35), viral fluid was administered at 2 µL into each nostril (1x10^5 TCID50/mouse). Three days later (day 38), nasal wash fluid and serum were collected. The viral titer in the nasal wash fluid, antigen-specific IgA in nasal wash fluid, and IgG in serum were measured by ELISA.

### (Results)

Fig. 8 shows the viral titer in nasal wash fluid (mean + standard error). Neither nasal administration nor subcutaneous administration of HA antigen vaccine alone showed any infection protective effect against the virus inoculated into the nasal cavity. In the group where HA antigen vaccine was co-administered with TY001 S-form, dose-dependent inhibition of viral infection was observed at TY001 S-form doses of 0.01-0.3 µg, with no virus detected in any individual at 0.1 and 0.3 µg.

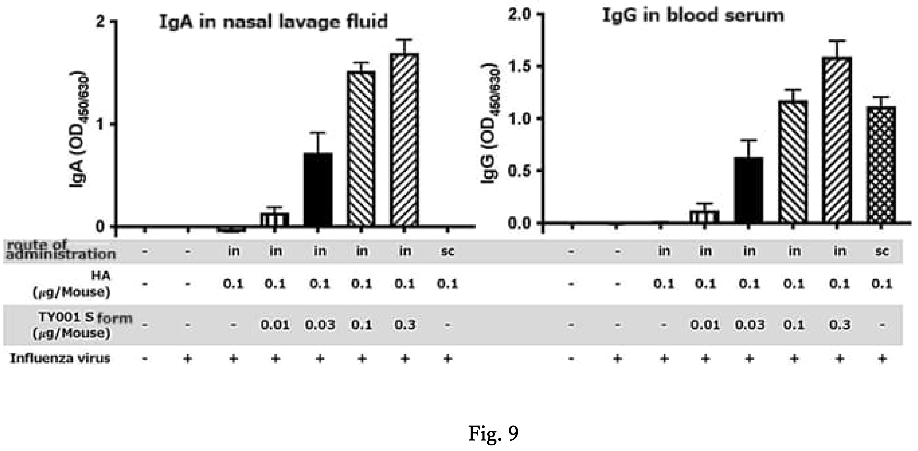

Fig. 9 shows the levels of antigen-specific IgA in nasal wash fluid and IgG in serum (mean + standard error). Nasal administration of HA antigen vaccine alone did not induce nasal IgA or serum IgG. Subcutaneous administration of HA antigen vaccine only induced serum IgG. In the group where HA antigen vaccine was co-administered with TY001 S-form, dose-dependent induction of nasal IgA and serum IgG was observed at TY001 S-form doses of 0.01-0.3 µg.

These results confirmed that the induction of antigen-specific IgA in the nasal cavity plays an important role in protecting against inoculated viruses.

### <Example 6: Evaluation of Nasal Adjuvant Effect of Improved TY001 S-form Formulation>

### <<Comparative Test of Influenza HA Antigen Vaccine Co-administered with TY001 S-form and Various Adjuvants>>

### (Method)

For the nasal administration test, 8-week-old female BALB/c mice (6 mice per group) were used. Influenza virus HA protein antigen: A/Guangdong-Maonan/SWL1536/2019(CNIC-1909)(H1N1) (Osaka University Microbial Disease Research Institute) was used. Vaccine formulations included HA 0.1 µg alone, and those co-administered intranasally with 1 µg of TY001 S-form (formulation containing 0.01% chondroitin sulfate sodium as an additive), Cholera Toxin B subunit (CTB) (Fujifilm Wako Pure Chemical Corporation), CpG ODN (ODN 1826) (InvivoGen), or Polyinosinic-polycytidylic acid (Poly(I:C)) (InvivoGen). Each vaccine solution was administered into the nasal cavity, 5 µL into each nostril (10 µL/mouse), twice at a 3-week interval. A group subcutaneously administered with HA 0.1 µg + Alum 2 mg was also used. Two weeks after the second administration (day 35), nasal wash fluid, bronchoalveolar lavage fluid, and serum were collected. Antigen-specific IgA in nasal wash fluid and bronchoalveolar lavage fluid, and IgG in serum and bronchoalveolar lavage fluid were measured by ELISA.

### (Results)

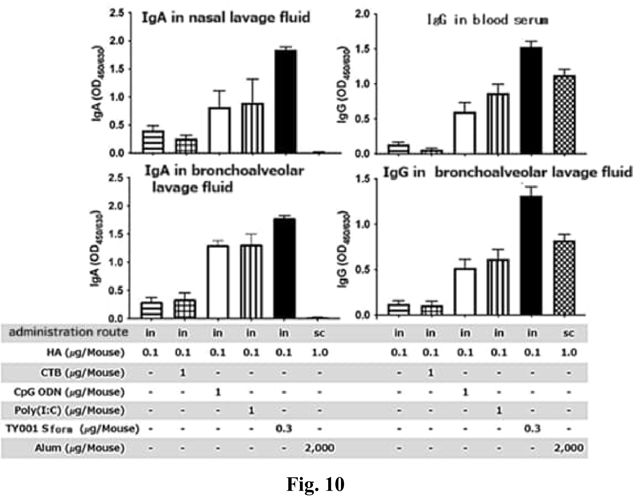

Fig. 10 shows the levels of antigen-specific IgA in nasal wash fluid and bronchoalveolar lavage fluid, and IgG in serum and bronchoalveolar lavage fluid (mean + standard error). Co-administration of HA antigen vaccine with TY001 S-form showed stronger antibody-inducing ability at a lower dose compared to any other adjuvant. Furthermore, IgA and IgG antibodies were also induced in the bronchoalveolar lavage fluid, confirming its performance as a mucosal adjuvant.

As described above, the effect of TLR2 and TLR7 dual agonists as mucosal adjuvants was demonstrated.

### [Industrial Applicability]

The present invention can be used as a mucosal adjuvant.

## Claims

1. A mucosal adjuvant comprising a compound represented by the following formula (I) or a pharmaceutically acceptable salt thereof:
A-L-B (I)
wherein,
A represents a structure having TLR7 agonist activity,
L represents a linker, and
B represents a structure having TLR2 agonist activity.

2. The adjuvant according to claim 1, wherein L has a structure represented by the following formula (L-1): 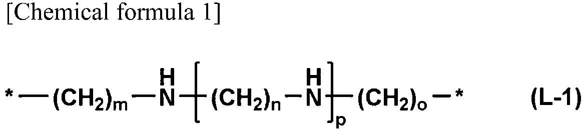 wherein,
m represents an integer from 0 to 4,
n represents an integer from 0 to 4,
o represents an integer from 0 to 4,
p represents an integer from 1 to 6, and
* indicates a bond to -NH-.

3. The adjuvant according to claim 1, wherein the compound represented by formula (I) is the compound TY001 represented by the following formula.

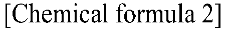

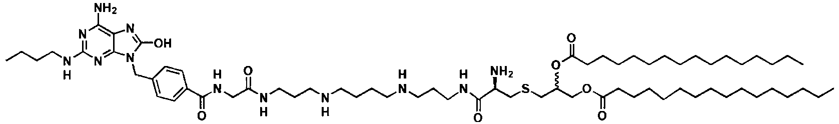

4. The adjuvant according to claim 3, wherein TY001 is a racemate, S-form, or R-form.

5. A vaccine composition comprising at least one antigen and the adjuvant according to any one of claims 1 to 4.

6. The composition according to claim 5, further comprising chondroitin sulfate and/or a salt thereof.

7. The composition according to claim 5, which is a liquid formulation, a spray formulation, a semi-solid formulation, or a solid formulation, wherein the semi-solid formulation and the solid formulation dissolve in body fluids and/or at body temperature.

8. The composition according to claim 5, for use in inducing humoral immunity.

9. The composition according to claim 5, for use in inducing the production of IgA antibodies in mucous membranes.

10. The composition according to claim 9, further for use in inducing the production of IgG antibodies in the blood.

11. The composition according to claim 5, which is a vaccine for infectious diseases.

12. The composition according to claim 11, wherein the antigen of the vaccine for infectious diseases is an antigen derived from a pathogenic virus, pathogenic bacterium, pathogenic fungus, or parasite.

13. The composition according to claim 12, wherein the antigen of the vaccine for infectious diseases is an antigen derived from influenza virus or coronavirus.

14. The composition according to claim 5, which is a vaccine for non-infectious diseases.

15. The composition according to claim 14, wherein the antigen of the vaccine for non-infectious diseases is an antigen derived from amyloid β, α-synuclein, prion, cholesteryl ester transfer protein, ApoB100, oxidized LDL, angiotensin I/II, glatiramer acetate, myelin basic protein, T cell receptor of MBP-specific T cells, insulin, GAD, T cell receptor of acetylcholine receptor-specific T cells, allergen, IL-5, cancer antigen, neoantigen, intoxicating substance, TNFα, HCG, GnRH, Ghrelin, or TRANCE/RANKL.

16. A compound represented by the following formula, or a pharmaceutically acceptable salt thereof.

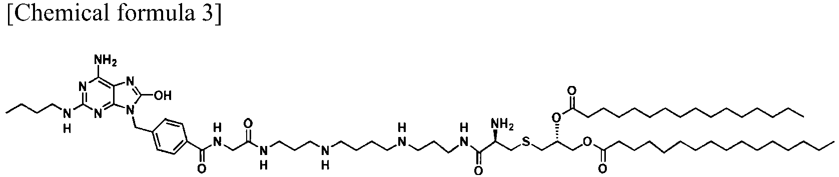

17. A compound represented by the following formula, or a pharmaceutically acceptable salt thereof.
